# EUROPÄISCHE PATENTSCHRIFT

(11) **EP 1 858 913 B1**
(45) Veröffentlichungstag und Bekanntmachung des Hinweises auf die Patenterteilung: **01.09.2010**
(21) Anmeldenummer: 06723211.6
(22) Anmeldetag: 03.03.2006
(51) Int. Cl.: C07K 5/08, A61K 38/06, A61P 35/00, A61P 25/28

(54) **PEPTID-MIMETIKA UND IHRE VERWENDUNG ALS INHIBITOREN DES 20S PROTEASOMS, 26S PROTEASOMS UND DES IMMUNOPROTEASOMS**
MIMETIC PEPTIDES AND THE USE THEREOF IN THE FORM OF 20S, 26S AND IMMUNOPROTEASOME INHIBITORS
PEPTIDES MIMETIQUES ET LEUR UTILISATION COMME INHIBITEURS DU PROTEASOME 20S, DU PROTEASOME 26S ET DE L'IMMUNOPROTEASOME

(30) Priorität: 03.03.2005 DE 102005009784
(43) Veröffentlichungstag der Anmeldung: 28.11.2007
(73) Patentinhaber: Technische Universität Darmstadt, 64287 Darmstadt (DE); Charité - Universitätsmedizin Berlin, 10117 Berlin (DE)
(72) Erfinder: SCHMIDT, Boris, 64293 Darmstadt (DE); UMBREEN, Sumaira, c/o Frau Brigitte Herdler, 64297 Darmstadt (DE); BRAUN, Hannes, 65719 Hofheim (DE); KLOETZEL, Peter-Michael, 12589 Berlin (DE); KUCKELKORN, Ulrike, 12621 Berlin (DE)
(74) Vertreter: Krauss, Jan
(86) Internationale Anmeldenummer: PCT/EP2006/001975
(87) Internationale Veröffentlichungsnummer: WO 2006/092326

(56) Entgegenhaltungen:
- EP-A- 1 454 627
- WO-A-00/35868
- WO-A-95/24914
- WO-A-95/25533
- WO-A-2004/089423
- WO-A-2006/021413
- SORG GERHARD ET AL: "Progress in the preparation of peptide aldehydes via polymer supported IBX oxidation and scavenging by threonyl resin." JOURNAL OF PEPTIDE SCIENCE : AN OFFICIAL PUBLICATION OF THE EUROPEAN PEPTIDE SOCIETY. MAR 2005, Bd. 11, Nr. 3, März 2005 (2005-03), Seiten 142-152, XP008052462 ISSN: 1075-2617
- IQBAL M ET AL: "Subsite requirements for peptide aldehyde inhibitors of human calpain I" BIOORGANIC & MEDICINAL CHEMISTRY LETTERS, OXFORD, GB, Bd. 7, Nr. 5, 4. März 1997 (1997-03-04), Seiten 539-544, XP004136061 ISSN: 0960-894X
- SAITO Y ET AL: "Isolation and characterization of possible target proteins responsible for neurite outgrowth induced by a tripeptide aldehyde in PC12H cells." BIOCHEMICAL AND BIOPHYSICAL RESEARCH COMMUNICATIONS. 15 APR 1992, Bd. 184, Nr. 1, 15. April 1992 (1992-04-15), Seiten 419-426, XP008052461 ISSN: 0006-291X
- PATENT ABSTRACTS OF JAPAN Bd. 010, Nr. 287 (C-375), 30. September 1986 (1986-09-30) & JP 61 103897 A (SUNTORY LTD; others: 01), 22. Mai 1986 (1986-05-22)
- BOILLOT F ET AL: "The Perseus Exobiology mission on MIR: behaviour of amino acids and peptides in Earth orbit." ORIGINS OF LIFE AND EVOLUTION OF THE BIOSPHERE : THE JOURNAL OF THE INTERNATIONAL SOCIETY FOR THE STUDY OF THE ORIGIN OF LIFE. AUG 2002, Bd. 32, Nr. 4, August 2002 (2002-08), Seiten 359-385, XP008052458 ISSN: 0169-6149
- TAGUCHI T ET AL: "Inhibition of DNA polymerases by tripeptide derivative protease inhibitors." BIOCHEMICAL AND BIOPHYSICAL RESEARCH COMMUNICATIONS. 30 JUN 1992, Bd. 185, Nr. 3, 30. Juni 1992 (1992-06-30), Seiten 1133-1140, XP008052561 ISSN: 0006-291X
- SAITO Y ET AL: "The structure-function relationship between peptide aldehyde derivatives on initiation of neurite outgrowth in PC12h cells." NEUROSCIENCE LETTERS. 27 NOV 1990, Bd. 120, Nr. 1, 27. November 1990 (1990-11-27), Seiten 1-4, XP002344897 ISSN: 0304-3940

## Beschreibung

### Beschreibung der Erfindung

Die vorliegende Erfindung betrifft peptid-mimetische Verbindungen, deren Synthese und Verwendung zur Inhibierung von Proteasomen und Induktion von Apoptose in Tumorzellen. Weiterhin betrifft die vorliegende Erfindung pharmazeutische Zusammensetzungen umfassend die Verbindungen und die Verwendung der Verbindungen zur Behandlung von Erkrankungen, insbesondere Krebserkrankungen und neurodegenerative Erkrankungen.

### Hintergrund der Erfindung

Das Gleichgewicht zwischen der Synthese und den Abbauprozessen von Proteinen ist essentiell zur Aufrechterhaltung der zellulären Homöostase. Zellen besitzen zwei hauptsächliche Stoffwechselwege der Proteindegradation. Ein großer Teil der Proteine wird entweder durch proteolytische Enzyme in Lysosomen oder über das Ubiquitin-Proteasom-System verdaut. Ein Ungleichgewicht zwischen der Proteinsynthese und den Proteinabbauprozessen führt zu einer Reihe von pathologischen Prozessen (1).

Die 26S Proteasomen sind Protease-Komplexe, die aus multiplen Untereinheiten aufgebaut sind und die die ATP-abhängige Degradation von poly-ubiquitinylierten Proteinen durchführen. Sie sind für den Großteil der nicht-lysosomalen Proteolyse in eukaryotischen Zellen verantwortlich. Sie bestehen aus dem proteolytischen 20S Proteasom-Kernpartilcel und tragen an einem oder beiden Enden einen Deckel, der aus den regulatorischen 19S Cap-Partikeln gebildet wird (2,3). Der 20S Kernpartikel ist ein zylindrischer Verband von 28 Untereinheiten, die in 4 gestapelten Heptamer-Ringen angeordnet sind. 2 Ringe werden von 7 Untereinheiten des α-Typs und 2 Ringe von 7 Untereinheiten des β-Typs gebildet (4,5). Die beiden inneren β-Ringe formen den zentralen Bereich des Zylinders und tragen die proteolytischen Zentren.. Im Gegensatz zu den prokaryotischen 20S Proteasomen, die aus 14 identischen alpha und 14 identischen proteolytisch aktiven Untereinheiten des β-Typs bestehen, besitzen eukaryotische 20S Proteasomen nur 3 proteolytisch aktive Untereinheiten pro β-Ring. Proteasomen gehören zur Familie der *N*-terminal nukleophilen Hydrolasen (6,7). Eine Stimulation von Säugetierzellen mit γ-Interferon verursacht den Austausch der 3 aktiven β-Untereinheiten β1, β2 und β5 durch die Immunhomologen β1i, β2i und β5i, was zur Bildung der Immunproteasomen führt, welche modifizierte Spaltmuster von Substratpeptiden generieren. Es wurde gezeigt, daß die funktionale Integrität der Proteasomen für eine Vielzahl von zellulären Funktionen unentbehrlich ist, wie für die metabolische Adaptation, Zelldifferenzierung, Zellzyklus-Kontrolle, Stressantwort, die Degradation von abnormalen Proteinen und die Generierung von Epitopen, die durch MHC Klasse I-Rezeptoren präsentiert werden (für einen Überblick: siehe (8,9)). Proteasomen sind ein bedeutender aber nicht ausschließlicher Erzeuger der antigenen Peptide (10,11).

Die Disregulation des Stoffwechselweges der Ubiquitin-Proteasom-Proteindegradation verursacht verschiedene Krankheiten im Menschen, wie zum Beispiel Krebs, neurodegenerative, Autoimmun- und metabolische Erkrankungen. Die Inhibierung der Proteasomen beeinflußt die Stabilität von vielen Proteinen, wie derjenigen, die an der Regulierung des Zellzyklus beteiligt sind. Daher sind selektive Inhibitoren der multikatalytischen proteasomalen Untereinheiten attraktive Ziele in der Entwicklung von Wirkstoffen (12).

Die meisten der mit proteasomalen Inhibitoren behandelten Zellen werden für die Apoptose sensibilisiert (13, 14). Interessanterweise sind Tumorzellen gewöhnlicherweise sensitiver gegenüber proteasomaler Inhibierung als normale Zellen. Gesunde Zellen unterliegen einem Zellzyklus-Arrest, wenn sie mit proteasomalen Inhibitoren behandelt werden, sind aber im Gegensatz zu Tumorzellen weniger anfällig für Apoptose (15, 16).

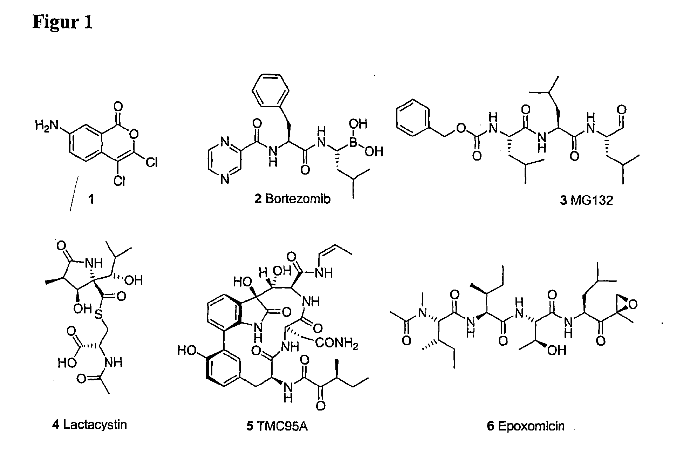

Bisher wurden verschiedene proteasomale Inhibitoren charakterisiert (siehe Figur 1). Man unterscheidet in selektive Inihibitoren **(4** Lactacystin, **5** TMC-95A, **6** Epoxomicin) und unselektive Inhibitoren **(1** Dichlorvinylester, **3** MG132) (17).

Der bedeutendste proteasomale Inhibitor ist Verbindung **2,** auch Bortezomib^{®} oder VELCADE^{™} (siehe Figur 1). Bortezomib^{®} wurde von der U.S. Food and Drug Administration (FDA) als verschreibungspflichtiger Arzneistoff zur Behandlung von multiplen Myeloma zugelassen (18-20).

Ein weiterer wichtiger proteasomaler Inhibitor ist MG132 (Verbindung **3** in Figur 1). Ein entscheidender Nachteil von MG132 ist seine mangelnde/geringe Selektivität in der Inhibierung von Proteosomen (1, 17, 22, 38).

Das Dokument WO 9524914 beschreibt zahlreiche ähnliche, aber nicht identische Peptidaldehyde wie die der vorliegenden Anmeldung. Die entscheidende Wirkungsverbesserung der erfindungsgemäßen Verbindungen entsteht durch die Asp(OR₄) Gruppe/Substituenten. Daraus resultiert eine selektivere Inhibition. Diese wesentliche Struktureinheit wird durch die Definition des Substituenten R2 (auf S. 13) nicht erfaßt.

Das Dokument Sorg et al. (2005) J. Peptide Science (11): 142-152. beschreibt die Synthese von Tri- und Tetrapeptidaldehyden an fester Phase. Die aktivsten Verbindungen der vorliegende Anmeldung lassen sich mit dieser Methode jedoch nicht darstellen, da dieser Ester unter den Reaktionsbedingungen instabil ist. Dementsprechend sind in der Publikation keine Tri- und Tetrapeptidaldehyde mit geschützter Glutar- und Asparaginsäure an Position 2 beschrieben. Eine biologische Wirkung der Substanzen wird nur für Caspasen beschrieben.

Das Dokument Iqbal et al. (1997) Bioorganic & Medicinal Chemistry Letters (7): 539-544. beschreibt die Inhibition von Calpain I durch Di- und Tripeptidaldehyde, vor allem mit P2 = Leu, Phe, Ser, Thr, Trp. Keines der aufgeführten Peptide trägt die polare Amino-säuren Glu, Asp, Asn, Gln oder Derivate davon in P2.

In dem Dokument Saito et al. (1992) Biochem. Biophys. Res. Comm. (184): 419-426. wird eine undefinierte Proteaseinhibition durch Tripeptidaldehyde der allgemeinen Formel Ac-Leu-Leu-X-CHO sowie Z-Leu-Leu-X-CHO auf unbekannte Proteasen beschrieben. Eine Identifizierung des aktiven Protease durch Affnitäts-Chromatagraphie verlief negativ, lediglich die Hemmung von Cathepsin L wurde bestätigt. 20S- oder 26S-Proteasom werden nicht genannt, das Proteasom ist auch keine sekretierte Protease.

Weiterhin beschreibt WO 96/13266 peptidische Boronsäure und -ester-Verbindungen, die sich als Inhibitoren der Proteosom-Funktion eignen.

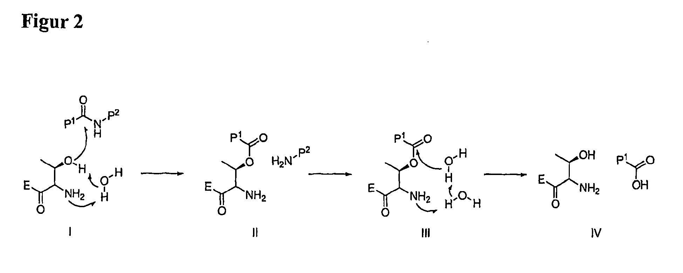

Die proteasomale Amid-Hydrolyse unterscheidet sich von der Amid-Hydrolye aller anderen Klassen von Proteasen. Die Besonderheit stellen dabei die N-terminale Threonine dar. Der Mechanismus ist in Figur 2 dargestellt. Durch die Analyse der Kristallstruktur des 20S Proteasoms wurde aufgedeckt, daß Thr1O^{γ} als das Nukleophil und die N-terminale Aminogruppe als der Acyl-Überträger fungiert (6). Kovalente Inhibitoren können im aktiven Zentrum binden, und zwar entweder über die Hydroxylgruppe des Thr1O^{γ} oder über den freien N-Terminus und Thr1O^{γ} gleichzeitig (für einen Überblick: siehe 17).

Effektive *in vivo* Inhibitoren des 20S Proteasoms benötigen daher eine hohe Selektivität und zugleich eine gute Penetrationsfähigkeit der Zellmembranen. Weiterhin könnten sie sich dadurch auszeichnen, daß sie kovalent an das N-terminale Threonin binden.

Aufgabe der vorliegenden Erfindung ist es daher, verbesserte Proteasom-Inhibitoren zu entwickeln, die sich insbesondere durch ihre Selektivität gegenüber dem Proteasom sowie ihre Irreversibilität auszeichnen und zur Penetration von Zellmembranen fähig sind.

Die Aufgabe wird erfmdungsgemäß durch das zur Verfügung stellen von Verbindungen mit der Formel 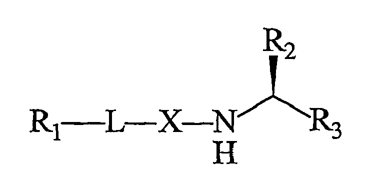 gelöst, worin R₁ bis R₅ und X unabhängig voneinander ausgewählt sind und worin
- R₁: Boc, Z, Ac oder H ist,
- L: Leu ist,
- X: Leu oder Asp(OR₄) ist,
- R₂: CH₂-CH(CH₃)₂ ist,
- R₃: CH₂-OH, CH=O, CH(OH)-C =C-Phenyl, CH(OH)-C(O)-NH-R₅ oder C(O)-C(O)-NH-R₅ ist,
- R₄: *t*-Butyl, Benzyl oder H ist,
- R₅: Benzyl, 3-Picolyl oder Phenyl ist,

Ausgenommen soll eine Verbindung sein, in der, wenn R₁ Z und X Leu ist, R₃ CH=O ist, und pharmazeutisch akzeptable Salze davon.

In einer bevorzugten Ausführungsform umfaßt die Erfindung Verbindungen, worin
- R₁: Boc oder Z,
- L: Leu,
- X: Asp(OR₄),
- R₂: CH₂-CH(CH₃)₂,
- R₃: CH₂-OH,
- R₄: *t*-Butyl,
ist.

In einer weiteren bevorzugten Ausführungsform umfaßt die Erfindung Verbindungen, worin
- R₁: Boc, Z oder Ac,
- L: Leu,
- X: Asp(OR₄),
- R₂: CH₂-CH(CH₃)₂,
- R₃: CH=O,
- R₄: *t*-Butyl oder Benzyl,
ist.

In einer weiteren bevorzugten Ausführungsform umfaßt die Erfindung Verbindungen, worin
- R₁: Z,
- L: Leu,
- X: Leu,
- R₂: CH₂-CH(CH₃)₂,
- R₃: C(O)-C(O)-NH-R₅,
- R₅: Benzyl, 3-Picolyl oder Phenyl
ist.

In einer weiteren bevorzugten Ausführungsform umfaßt die Erfindung Verbindungen, worin
- R₁: Z,
- L: Leu,
- X: Leu,
- R₂: CH₂-CH(CH₃)₂,
- R₃: CH(OH)-C(O)-NH-R5,
- R₅: Phenyl
ist.

In einer weiteren bevorzugten Ausführungsform umfaßt die Erfindung Verbindungen, worin
- R₁: Z,
- L: Leu,
- X: Leu,
- R₂: CH₂-CH(CH₃)₂,
- R₃: CH(OH)-C≡C-Phenyl,
ist.

Die Erfindung stellt weiterhin Verfahren zur Herstellung der erfindungsgemäßen Verbindungen zur Verfügung. Ein solches Verfahren umfaßt bevorzugt einen Oxidations- oder einen Reduktionsschritt. Bevorzugterweise ist das erfindungsgemäße Verfahren **dadurch gekennzeichnet, daß** die Oxidation durch hypervalente Iodreagentien erfolgt.

Das erfindungsgemäße Verfahren umfaßt dabei bevorzugt die Umsetzung von Aminoalkoholen zu Peptidmimetika **(7-12)** mit anschließender Oxidation zu Peptidaldehyden **(13-18),** z.B. durch hypervalente Iodreagentien. Die Synthese ist ebenfalls möglich durch die Reduktion von derivatisierten Aminosäureestern zu den entsprechenden Peptidaldehyden.

Die Synthese der erfindungsgemäßen Verbindungen **7-18,** die ausgehend von Verbindung **3** (MG132) als Leit-Struktur erfolgte, wurde basierend auf den etablierten Substrat-Präferenzen von β-Sekretase (23) durch Standardmethoden durchgeführt.. Die Synthese ist im Schema I dargestellt (siehe auch Beispiel 1). Der Kondensation von kommerziell erhältlichen, geschützten Dipeptiden und Aminosäuren mit kommerziell erhältlichen Aminoalkoholen folgte die Oxidation zu den Aldehyden durch IBX in DMSO (Schema I).

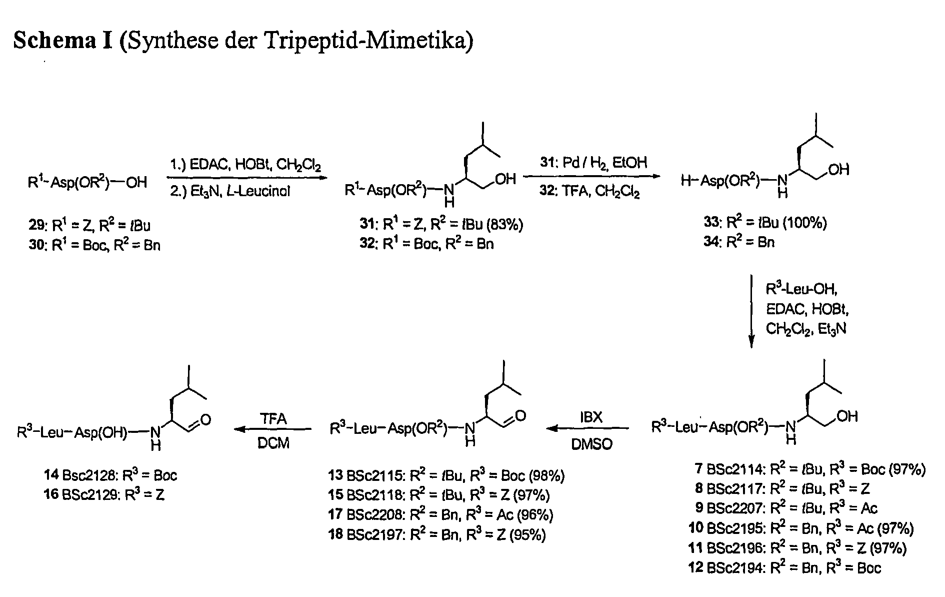

Die Intermediate, d.h. die Alkohol-Derivate **7-12,** und die Tripeptidaldehyde **13-18** wurden auf ihre Fähigkeit zur Enzym-Inhibierung untersucht. Die Inhibierung der β-Sekretase war eher schwach ausgeprägt (IC₅₀ > 200 µM, nicht gezeigte Ergebnisse), aber verschiedene Verbindungen erwiesen sich als potente Inhibitoren des 20S Proteasoms.

Peptidaldehyde weisen im allgemeinen keine Selektivität in der Inhibierung von Enzymen auf. Daher wurden verschiedene Gruppen auf ihre Fähigkeit getestet, Threonin-Proteasen zu inhibieren.

Der unselektive Dichlorvinylester **1** (siehe Figur 1), welcher leicht mit allen möglichen Nukleophilen, wie zum Beispiel Cystein, Serin und letztendlich auch Threonin, reagiert, diente als weitere Leitstruktur für Synthesen von erfindungsgemäßen Verbindungen. Es wurde außerdem das Ziel verfolgt, die inhärente Überaktivierung dieser Verbindung zu reduzieren. Die "Entfernung" der Acylgruppe aus **1** könnte die unspezifische Hydrolyse durch ubiquitäre Nukleophile reduzieren und resultiert in recht stabilen Dichlorvinylethem (28). Die resultierenden Ether, die erfindungsgemäßen Verbindungen **19-20** (zur Synthese siehe Schema II sowie Beispiel 1), tolerieren eine saure Umgebung, aber werden leicht bei pH 11 hydrolysiert und in α-Chloracetate umgewandelt, welche wiederum mit Nukleophilen reagieren. Diese duale Reaktivität, welche in einer Kaskaden-artigen Reaktion bereitgestellt wird, entspricht den spezifischen Anforderungen an einen N-terminalen Threonin-Protease-Inhibitor.

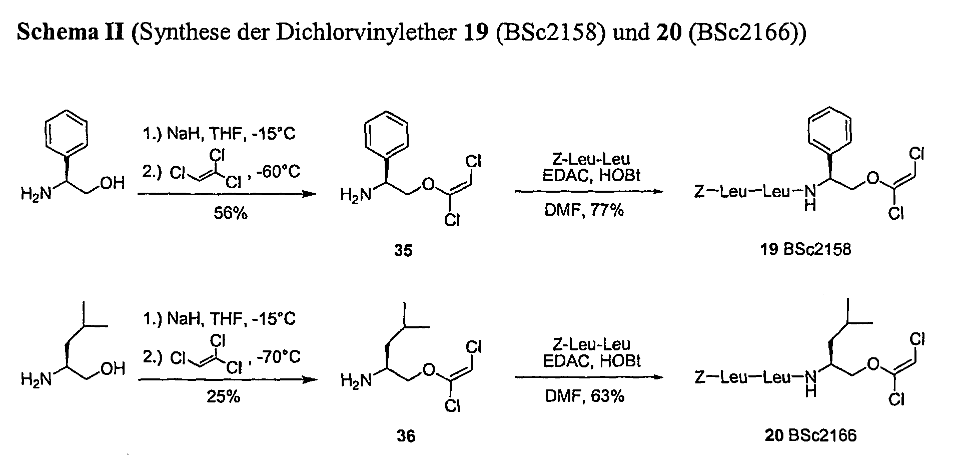

Eine analoge duale Reaktivität kann bei Propargyl-Ketonen beobachtet werden. Eine ähnliche Verbindung wurde synthetisiert, aber leider widerstand der Alkohol **21** (Schema III) der Oxidation zum gewünschten Keton.

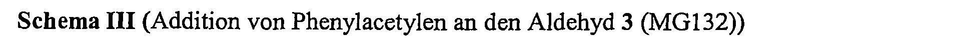

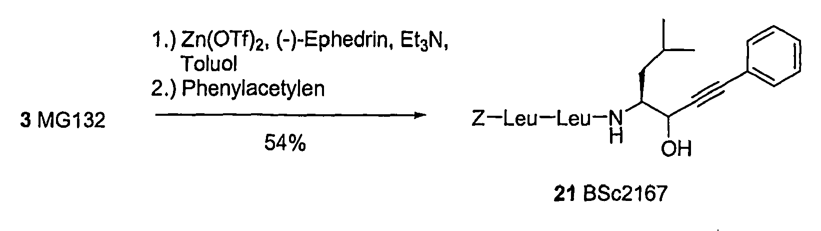

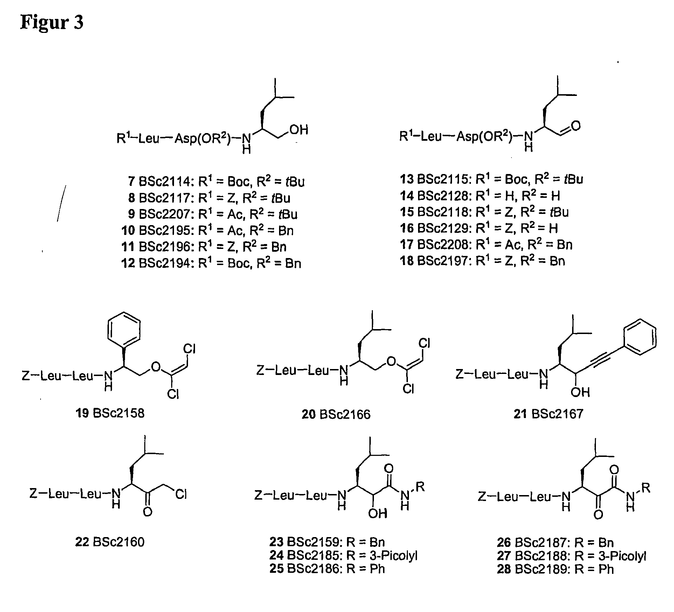

Daher richtete sich der weitere Fokus auf Übergangszustand-Mimetika und Inhibitoren. Leit-Strukturen, wie Statine (38), α-Ketoamide und Chlormethylketone, sind in der Inhibierung von Proteasen gut etabliert. Die Kombination von diesen Strukturen mit einem β-selektiven Tripeptid führte zu den erfindungsgemäßen Verbindungen **22-28** (Strukturen von **22-28,** siehe Figur 3). Verbindung **22** wurde aus dem kommerziellen Z-Leu-Leu und Chloromethylleucin präpariert (Schema IV und Beispiel 1).

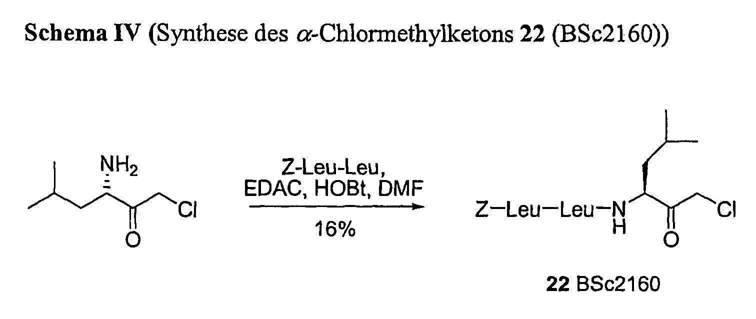

Die erfindungsgemäßen Verbindungen **23-25** wurden durch eine Passerini-Reaktion von MG132 (3) mit drei Isonitrilen erhalten. Die nachfolgende Oxidation durch IBX in DMSO lieferte die α-Ketoamide **26-28** (Schema V und Beispiel 1).

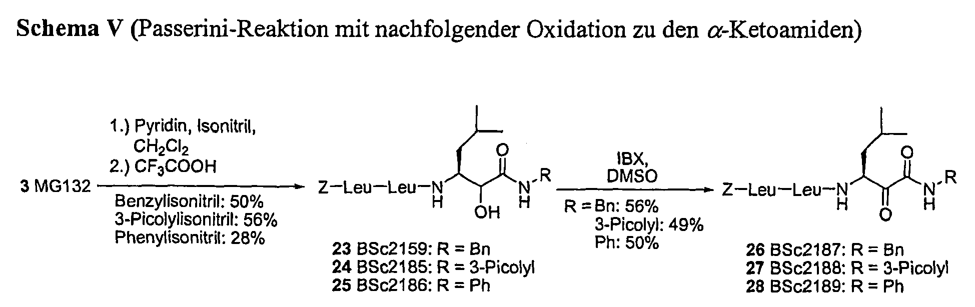

Proteasome sind in eine Reihe von verschiedenen zellulären Prozessen involviert. Sie sind bedeutsam für die Kontrolle des Zellzyklus und schützen Zellen vor Apotose durch das Aufrechterhalten des Gleichgewichts von anti-apoptotischen und pro-apoptotischen Proteinen (9, 31, 32). Das Interesse an potenten und spezifischen Inhibitoren, die als potenzielle Wirkstoffe gegen Krebs oder neoplastisches Wachstum verwendet werden können, ist sehr hoch.

In der vorliegenden Erfindung wird über die Synthese von Inhibitoren berichtet, die auf dem proteasomalen Peptid-Inhibitor MG132 basieren, welcher ein potenter, aber unspezifischer Inhibitor ist. Seitenketten-Modifizierungen dieses Tripeptids sollten zu einer höheren Potenz, Selektivität und orts-spezifischen Inhibierung des 20S Proteasoms führen. Diese Annahme basierte auf einer Reihe von bekannten und potenten peptidischen Inhibitoren (17, 33, 34, 35).

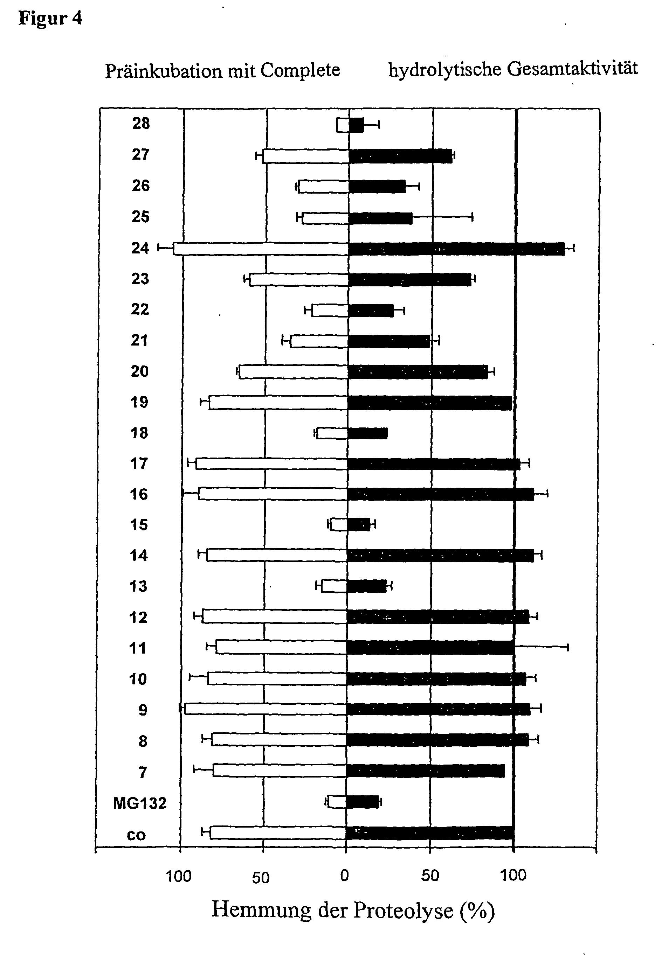

Alle erfindungsgemäßen Verbindungen wurden auf ihre inhibitorische Kapazität in Zell-Lysaten getestet. Daher wurden Serin-, Cystein- und Metall-Proteasen durch den Protease-Inhibitor-Cocktail Complete (Roche) während der Tests mit den synthetisierten Mimetika blockiert. Die Proteolyse des hydrophoben Substrats Suc-LLVY-AMC wurde von 10 der untersuchten erfindungsgemäßen Verbindungen verringert (siehe auch Beispiel 5, Figur 4).

Die spezifische Inhibierung einer einzelnen katalytischen Stelle ist von speziellem Interesse für die Wirkstoff-Entwicklung. Daher wurde die Inhibierung der verschiedenen proteasomalen Aktivitäten des Proteasoms analysiert (siehe auch Beispiel 6). Die verschiedenen Spaltungs-Präferenzen des Proteasoms wurden durch spezifische Substrate für die hydrophoben (Chymotrypsin-artigen), die Trypsin-artigen und die Caspase-artigen Aktivitäten von isolierten Proteasomen bestimmt. 12 von 22 erfindungsgemäßen Derivaten inhibierten proteasomale Aktivitäten mit IC₅₀-Werten unter 10 µM (siehe Tabelle 1). Die peptidischen Derivate **13** und **15** inhibierten alle der proteasomalen hydrolytischen Aktivitäten, wohingegen vier Verbindungen (**18, 25, 26** und **27)** die chymotryptischen und die Caspase-artigen Stellen inhibierten.

Jedoch war ein Zweck dieser Untersuchung auch die Identifizierung von vollständig selektiven Inhibitoren der proteasomalen Aktivität. Der tripeptidische Alkohol 7 (und mit niedrigerer Potenz Verbindung **8)** reduzierte spezifisch die Trypsin-artige Aktivität und die Verbindungen **16, 21, 22** und **28** resultierten in einer exklusiven Reduzierung der chymotryptischen Aktivität. Bemerkenswerterweise haben die potentesten der neuen Inhibitoren IC₅₀-Werte unter 100 nM **(7, 15, 28).** Diese liegen im Bereich der neuen proteasomalen Inhibitoren, die sich zur Zeit in klinischen Phasen befinden (33).

Bemerkenswerterweise ist der Tetrapeptid-Inhibitor PSI (Z-Ile-Glu(O^{t}Bu)Ala-Leu-CHO) (36) strukturell verwandt mit der erfmdungsgemäßen Verbindung **15** (Z-Leu-Asp(O^{t}Bu)Leu-CHO), die zu den stärksten Inhibitoren zählt (IC₅₀ unter 60 nM).

Weiterhin zeigte **15** eine niedrige Toxizität und war in der Lage, Zellmembranen zu durchdringen.

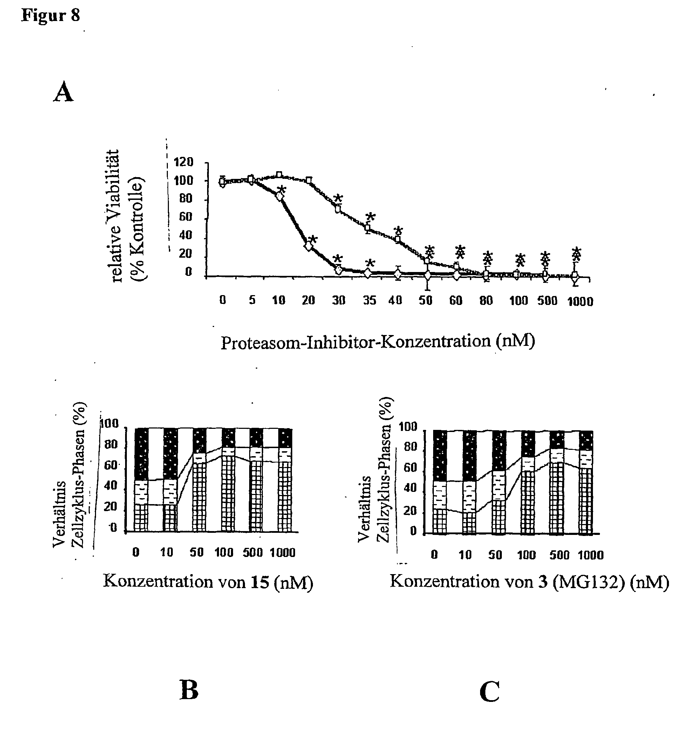

Der Vergleich der erfindungsgemäßen Inhibitoren zeigt, daß die Liganden-Seitenketten die hauptsächlichen Beiträge zu den spezifischen und festen Interaktionen mit den verschiedenen protolytischen katalytischen Zentren liefern (Figur 8 und Beispiel10). Ähnliche Beobachtungen wurden für die Alkohol-Derivate gemacht, wovon Verbindung 7 effektiver ist als die anderen sechs. Weiterhin wurden sehr potente Inhibitoren im Chlormethylketon (Verbindung **22)** und in Verbindungen **25-28** identifiziert.

Tumorzellen mit ihrem beschleunigten neoplastischen Wachstum sind oftmals sensitiver gegenüber proteasomalen Inhibitoren im Vergleich zu normalen Zellen. Der klinisch erprobte proteasomale Inhibitor Bortezomib^{®} verursachte Wachstumsarrest und Apoptose in sensitiven Tumorzellen, wohingegen "normale" Zellen hörere Inhibitorzellen tolerieren (37). Die Restriktion auf Myeloma-Tumore könnte durch spezifischere Inhibitoren, wie PSI, welcher Angiogenese blockiert und daher das Wachstum von soliden Tumoren moduliert (36), überwunden werden. Die Differenzen in zellulären Eigenschaften und die vorhersagbaren Resistenz-Mechanismen erfordern eine kontinuierliche Entwicklung von neuen proteasomalen Inhibitoren. Effiziente Zell-Permeation, Stabilität in wäßrigen Systemen und potente Induktion von zellulären Ereignissen sind alle obligatorisch für klinische Anwendungen.

Daher wurde die Permeations-Fähigkeit der erfmdungsgemäßen Verbindungen und ihr *in vivo* Einfluß auf Proteasomen getestet und eine Akkumulierung von poly-ubiquitinylierten Proteinen in kultivierten Zellen beobachtet. Mehr als 50%ige Reduktion der intrazellulären proteasomalen Aktivität wurde für 5 der Inhibitoren **(15, 22, 25, 26, 28)** bereits nach einer 20-stündigen Inkubation beobachtet. Bemerkenswerterweise war die proteasomale Aktivität sogar auf 10% bei Anwesenheit von **15, 26** und **28** reduziert. Schwache Inhibitoren haben einen geringen Einfluß auf die zelluläre Funktion. Die Ergebnisse der vorliegenden Erfindung zeigen Potenz, Membran-Permeation und ausreichende Stabilität während der Inkubationsperioden für die Inhibitoren **15, 22, 25, 26** und **28** an. Die zelluläre proteasomale Aktivität wird eindeutig reduziert und von einer starken Induktion von Apoptose nach 20-stündiger Behandlung mit 1 µM der Inhibitoren **(15, 26, 28)** begleitet. Die bekannte verstärkte Sensitivität von Tumorzellen gegenüber proteosomaler Inhibierung wurde für Inhibitor **15** und **28** bestätigt. Überraschenderweise wurde eine starke Induktion der Apoptose in Zellen, die mit Verbindung **7** präinkubiert wurden, beobachtet, welches die Trypsin-artige Aktivität auf exklusive Weise inhibierte. Diese Ergebnisse deuten darauf hin, daß die Trypsin-artige Aktivität insbesondere für anti-apoptotische Prozesse bedeutsam ist.

Erfindungsgemäß können die Verbindungen zur Induktion von Apoptose in Zellen verwendet werden.

Weiterhin können die erfindungsgemäßen Verbindungen zur Inhibierung der proteolytischen Aktivität von 20S Proteasom, 26S Proteasom und/oder Immunoproteasom verwendet werden. Dabei werden sie zur *in vitro, in vivo* und/oder intrazellulären Inhibierung verwendet.

Bevorzugt wird dabei spezifisch die Trypsin-artige Aktivität von 20S Proteasom, 26S Proteasom und/oder Immunoproteasom inhibiert.

Weiterhin bevorzugt wird spezifisch die Chymotrypsin-artige Aktivität von 20S Proteasom, 26S Proteasom und/oder Immunoproteasom inhibiert.

Es können auch bevorzugt gleichzeitig die Chymotrypsin-artigen, Trypsin-artigen und Caspase-artigen Aktivitäten von 20S Proteasom, 26S Proteasom und/oder Immunoproteasom inhibiert werden.

Es ist weiterhin gemäß der vorliegenden Erfindung bevorzugt, die Verbindungen zur Behandlung von Erkrankungen einzusetzen, wie zur Behandlung folgender Indikationsgebiete:

### - Neurologie

Inhibitionen oder Fehlfunktionen von Proteasomen sind assoziiert mit der Entstehung von Alzheimer, Parkinson und der Pick-Krankheit. Proteasomen sind beteiligt bei Amylotropher Lateraler Sklerose (ALS), bei Erkrankungen von Motorneuronen, der Polyglutamin-Krankheit und muskulären Dystrophien.

### - Tumorerkrankungen

Proteasome spielen eine Rolle in maligner Transformation, Regulation von Zellzyklus, Inhibition bzw. Exekution von Apoptose, Abbau von diversen Tumorsuppressor-Produkten (APC, p53, Rb), Abbau von Proto-Onkogenen (Raf, Myc, Myb, Rel, Src etc.), Störungen in der Zellzyklus-Regulation. Proteasomen sind für den Abbau von Zyklinen, CDK's und deren Inhibitoren zuständig; eine Hemmung von Proteasomen führt in den meisten Fällen zu einem Zyklusarrest.

### - Virale Erkrankungen

Die Präsentation von viralen Antigenen erfordert deren Generation durch Proteasomen: z.B. HCMV, Hepatitis (HCV und HBV), Herpes (HVP) u.a. Darüber hinaus ist für Coxsackie (CVB) und HCMV eine Beteiligung von Proteasomen bei der Virusreplikation wahrscheinlich (39, 40)

### - Endokrinologie

Glucokorticoide regulieren z.B. eine proteasomale alpha-Untereinheit rauf. Der Abbau von Proteinen bei der Thyroxin-Bildung erfolgt durch Proteasomen.

### - Immunologie

Es gibt eine Beteiligung von Proteasomen bei Entzündungsreaktionen (MHC Klasse I Liganden, Induktion von Immunoproteasomen durch Zytokine). Weiterhin besteht eine mögliche Rolle bei der Entstehung und dem Verlauf von Autoimmunerkrankungen. Eine Bestimmung von Proteasomen-Antikörpem ist im Serum von SLE-, Sjögrensyndrom- und Polymyositis-Patienten möglich, z.T. ist ein Nachweis von zirkulierendem, freigesetztem Proteasom im Serum dieser Patienten möglich (41).

Die vorliegende Erfindung stellt weiterhin pharmazeutische Zusammensetzungen zur Verfügung, umfassend eine oder mehrere der erfindungsgemäßen Verbindungen oder ein pharmazeutisch akzeptables Salz davon sowie pharmazeutisch akzeptable Träger und/oder Hilfsstoffe. Derartige pharmazeutisch akzeptablen Träger und Hilfsstoffe sind dem Fachmann bekannt.

Die pharmazeutischen Zusammensetzung gemäß der Erfindung sind **dadurch gekennzeichnet, daß** die Verbindung(en) in einer Menge vorliegt, so daß ein Konzentrationsbereich von bevorzugt 0,001 bis 100 µM, weiter bevorzugt von 0,01 bis 10 µM bei der Behandlung *in vivo* vorliegt.

Sie sind weiterhin **dadurch gekennzeichnet, daß** die Verbindung(en) in einer Menge vorliegt, die effektiv die Proteasom-Funktion in einer Zelle oder einem Säugetier hemmt.

Die vorliegende Erfindung stellt weiterhin pharmazeutische Zusammensetzungen zur Inhibierung des Wachstums einer Krebszelle zur Verfügung, umfassend in Kontakt bringen einer Zelle mit einer erfindungsgemäßen Verbindung oder mit einer erfindungsgemäßen pharmazeutischen Zusammensetzung.

Die vorliegende Erfindung soll durch die folgenden Beispiele unter Bezugnahme auf die beigefügten Figuren verdeutlicht werden.

### Figuren

In den Figuren zeigen
- **Figur 1**: Bekannte Inhibitoren von Serin- and Threonin-Proteasen.
- **Figur 2**: Mechanismus der Hydrolyse durch Threonin-Proteasen.
- **Figur 3**: Formeln der Verbindungen **7-28.**
Bei den Verbindungen 7-18 handelt es sich um die Peptid-Mimetika der vorliegenden Erfindung, die als Inhibitoren des 20S-Proteasoms verwendet werden können.
- **Figur 4**: Hemmung der Proteolyse in Zell-Lysaten durch Addition der Verbindungen **7-28** und MG132. 10 µM der Verbindungen **7-28** bzw. MG132 wurden zu geklärten Lysaten addiert und für 30 min präinkubiert, ehe der Proteolyse-Assay durchgeführt wurde. Parallel dazu wurde Lysat mit der kommerziellen Inhibitor-Mischung *Complete* (Roche), die die meisten der cytosolischen Serin- und Aspartat-Proteasen nicht aber Proteasomen inhibiert, für 30 min präinkubiert. Dieser partiellen Inaktivierung folgte dann die Inkubation mit den Verbindungen 7-28 und MG132. Die proteolytische Aktivität wurde in 10 µl der Lysate durch Addition von LLVY-AMC (100 µl, 50 µM in 20 mM Tris, pH 7,2, 1 mM EDTA, 1 mM DTT) bestimmt. Das AMC, das in nicht inhibiertem Lysat freigesetzt wurde, wurde auf 100% gesetzt. MG132 diente als Inhibitions-Kontrolle.
- **Figur 5**: Viabilität von HeLa-Zellen nach der Inkubation mit den Verbindungen **7-28.**
**A,** Die Viabilität von HeLa-Zellen, die mit den Verbindungen **7-28** und MG132 (1 µM) inkubiert wurden, wurde durch Kristall-Violett-Färbung nach 20 h bestimmt.
**B,** Die Viabilität von HeLa-Zellen ist abhängig von der Inhibitorkonzentration. HeLa-Zellen wurden für 20 Stunden bei Anwesenheit steigender Konzentrationen (10 bis 10000 nM) der Verbindungen **(7, 13, 15, 18, 20-22, 24-28)** und MG 132 kultiviert. Das Überleben der Zellen wurde durch Kristall-Violett-Färbung bestimmt.

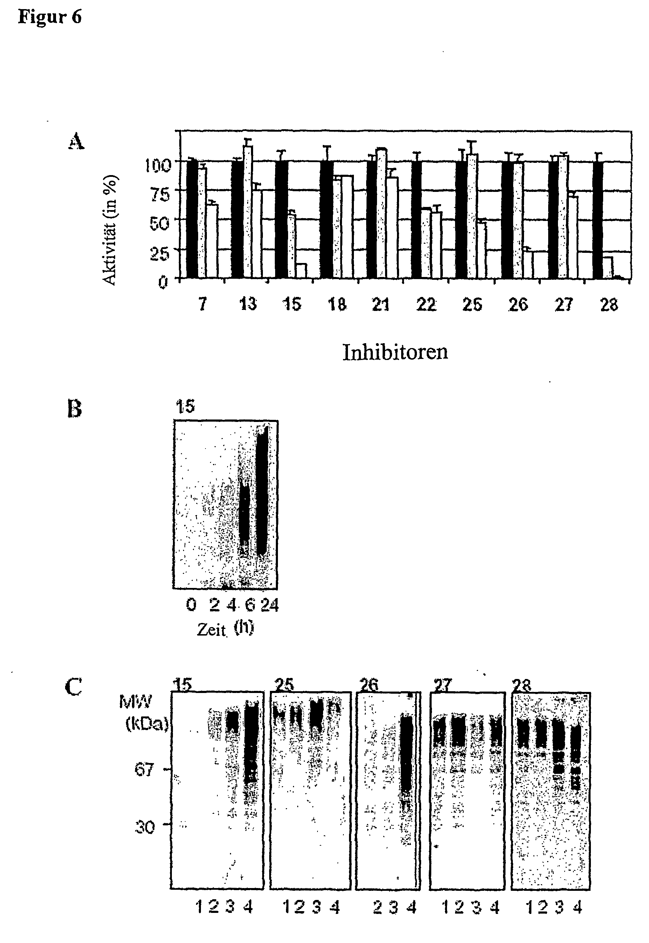

- **Figur 6**: Inhibierung der Proteasomen in Zellen.
**A,** HeLa-Zellen wurden für 24 Stunden bei Anwesenheit der Inhibitoren kultiviert. Die Proteinkonzentration wurde nach der Zell-Lyse nach Bradford bestimmt, um die proteasomalen Aktivitäten auf die unterschiedlichen Zellmengen zu normalisieren. Danach wurde *Complete* (Roche) zu allen Lysaten addiert und die proteasomale Aktivität durch die Hydrolyse von Suc-LLVY-AMC bestimmt.
**B,** HeLa-Zellen wurden mit 1 µM der Verbindung **15** for 2, 4, 6 und 24 Stunden inkubiert. Zellen wurden lysiert und Proteine durch SDS-PAGE auf einem 10%igen Gel getrennt und auf eine PVDF-Membran geblottet. Die Akkumulierung der polyubiquitinylierten Proteine wurde in einem Western blot durch einen Anti-Ubiquitin-Antikörper (DAKO) detektiert.
**C,** HeLa-Zellen wurden mit steigenden Konzentrationen der Inhibitoren **15, 25, 26, 27,** und **28** für 24 Stunden behandelt *(Bahn 1:* ohne Inhibitor;. *Bahn* 2: 10 nM; *Bahn* 3: 100 nM und *Bahn 4:* 1 µM des angegebenen Inhibitors). Zellen wurden lysiert, Proteine wurden in 15% Gelen aufgetrennt und die Akkumulierung der polyubiquitinylierten Proteine durch Western blots kontrolliert.

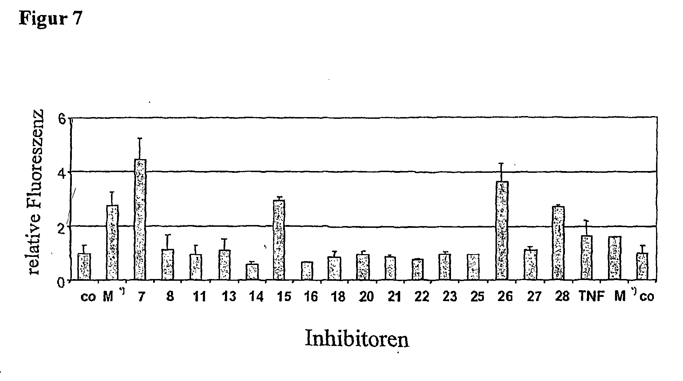

- **Figur 7**: Die Proteasomen-Inhibierung durch die Verbindungen **7, 15, 26** und **28** führt zur Induktion von Apoptose.
HeLa-Zellen, die für 20 Stunden In Anwesenheit der angegebenen Inhibitoren kultiviert wurden, wurden anschließend für 2 Stunden mit Caspase-Substrat (Apo-One; Promega) inkubiert. Die Aktivierung von Caspasen 3/7 wurde bei 538 nm (Anregung bei 485 nm) gemessen. Behandlung der Zellen mit TNFα oder mit MG132 diente als Kontrolle.
- **Figur 8**: Humane Melanoma-Zellen zeigen eine hohe Empfindlichkeit gegenüber Verbindung **15** und Untersuchungen zum Auslösen eines Zellzyklusarrestes durch Verbindung **15.**
**A,** Humane Melanoma-Zellen (MeWo) wurden mit steigenden Konzentrationen der Verbindungen **15** oder **3** (MG132) für 72 Stunden behandelt. Die Vitalität der Zellen nach der Behandlung mit Verbindung **15** (-◆-) und mit MG132 (-□-) wurde durch eine Kristallviolett-Färbung ermittelt.
**B und C,** MeWo Zellen wurden mit den Verbindungen **15** and **3** (MG132) für 24 Stunden ko-kultiviert. Die Zellen wurden anschließend in 70% Ethanol fixiert und mit RNAse A behandelt. Die DNA wurde durch Propidiumjodid (5 µg/ml) gefärbt und mittels FACS analysiert (FACS Calibur flow cytometer; Beckton Dickinson). Die statistische Signifikanz wurde durch den Chi-Square-Test ermittelt. Die relative Verteilung der Zellen, die sich in der G1- (schwarz), in der S- (weiß) bzw. in der G2 Phase (schraffiert) des Zellzyklus befanden wird für beide untersuchten Verbindungen gezeigt **(15** in **B** und MG132 in **C).**

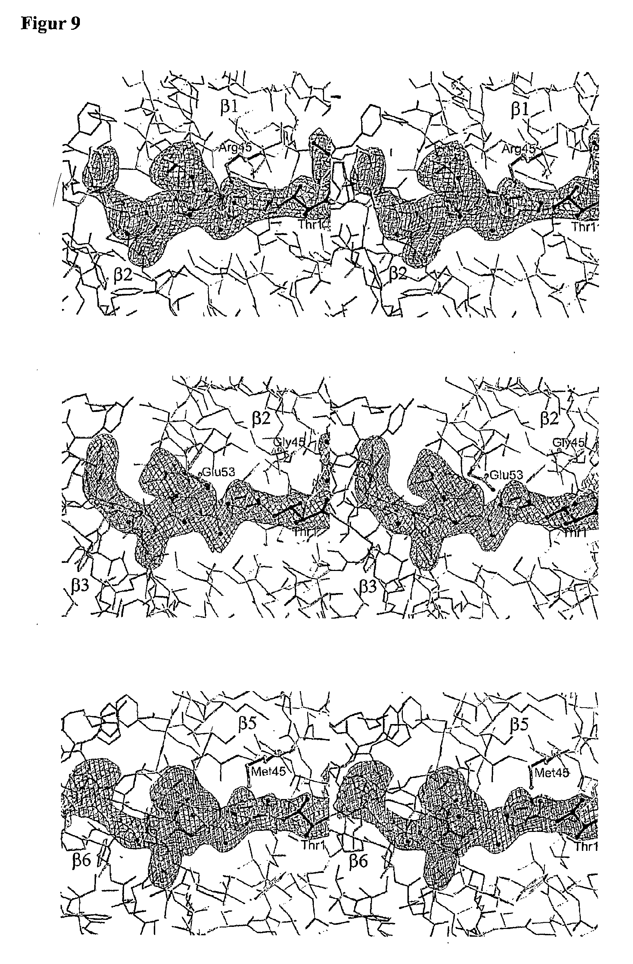

- **Figur 9**: Kristallstruktur von Verbindung **15** mit einem 20S-Proteasom.
Stereoansicht eines 30 Ǻ-Sektors der Kristallstruktur der aktiven Zentren von den β1 (a), β2 (b) und β5 (c)-Untereinheiten des 20S Proteasoms von Hefe in Komplex mit Verbindung **15.** Der Aldehyd **15** ist für jede Unterheit in seiner unbearbeiteten Elektronendichte dargestellt. Das Thr1 im aktiven Zentrum ist Schwarz hervorgehoben. Die kovalente Bindung zwischen **15** and Thr1Oγ ist abgebildet. Die Reste, welche für den Charakter der S1-Stelle insbesondere verantwortlich sind, sind in grau gezeichnet.

### Beispiele

### Beispiel 1: Synthese der Verbindungen

### A) Allgemeines.

Die ¹H- und ¹³C-NMR-Spektren wurden an einem Bruker AC 300 Spektrometer bei 300 MHz aufgenommen (75 MHz). Die chemischen Verschiebungen werden ppm-Werte feldabwärts von Me₄Si (TMS) angegeben. Massenspektrometrie wurde an einem Bruker-Franzen Esquire LC Massenspektrometer durchgeführt. Flash-Säulenchromatographie wurde unter der Verwendung von Merck Silicagel 60 (40-63 und 15-40 µm) und 60G (5-40 µm) durchgeführt. Dünnschichtchromatographie (TLC) wurde unter der Verwendung von Aluminiumplatten, die mit Silicagel 60 F254 (0,2 mm; E. Merck) beschichtet sind, durchgeführt. Die chromatographischen Flecken wurden durch UV und/oder Besprühen mit einer sauren, ethanolischen Lösung von p-Anisaldehyd oder einer ethanolischen Lösung von Ninhydrin mit nachfolgendem Erhitzen sichtbar gemacht. Für präparative Dünnschichtchromatographie wurden mit Silicagel 60 F254 (2,0 mm; E. Merck) beschichtete Platten verwendet. Aminosäure-Derivative wurden von Fluka Chemie (Schweiz), NovaBiochem (Schweiz) oder Bachem (Schweiz) erworben. THF wurde getrocknet und destilliert mit Natrium und Benzophenon. DMF wurde über 3Å-Molekularsieb gelagert. Alle anderen kommerziellen Chemikalien wurden ohne weitere Reinigung verwendet.

### B) Verbindung 7 (BSc 2114).

Ethyl-3-(3'-dimethylaminopropyl)carbodiimidhydrochlorid (EDAC, 191 mg, 1,0 mmol) und *N*-Hydroxybenzotriazolhydrate (HOBt, 183 mg, 1,2 mmol) wurden zu einer Lösung von Z-Asp(OtBu)-OH (**29**, 323 mg, 1,0 mmol), das in CH₂Cl₂ (10 mL) gelöst war, addiert. Die resultierende Mischung wurde bei Umgebungstemperatur für 5 min gerührt, danach mit *L-*Leucinol (117 mg, 1,0 mmol) und Triethylamin (151 mg, 1,5 mmol) für 24 h behandelt. CH₂Cl₂ (20 mL) wurde addiert und die Lösung mit HCl (0,1 N, 5 x 30 mL), NaOH (0,1 N, 3 x 30 mL), gesättigter NaCl-Lösung (1 x 30 mL) gewaschen, über Na₂SO₄ getrocknet und konzentriert, um Produkt **31** zu erhalten (350 mg, 83%). Eine Lösung von **31** (422 mg, 0,8 mmol) in Ethanol (10 mL, abs.) wurde mit Pd/C-Katalysator (10% Kohlenstoff, 100 mg) unter Wasserstoffatmosphäre bei Raumtemperatur behandelt. Die Suspension wurde nach 3 h filtriert und das Lösungsmittel im Vakuum entfernt, um Verbindung **33** zu erhalten (228 mg, 100%). EDAC (157 mg, 0,82 mmol) und HOBt (132 mg, 0,98 mmol) wurden zu der Lösung von Boc-Leu-OH (189 mg, 0,82 mmol) in CH₂Cl₂ (10 mL) addiert. Die resultierende Mischung wurde bei Umgebungstemperatur für 5 min gerührt, danach mit Verbindung **33** (228 mg, 0,82 mmol) und Triethylamin (124 mg, 1,23 mmol) für 24 h behandelt. DCM (20 mL) wurde addiert und die Lösung mit HCl (0,1 N, 5 x 30 mL), NaOH (0.1 N, 3 x 30 mL) und gesättigter NaCl-Lösung (1 x 30 mL) gewaschen. Das Lösungsmittel wurde nach dem Trocknen (Na₂SO₄) im Vakuum entfernt, um Verbindung **7** (BSc2114) zu erhalten (400 mg, 97%).
**¹H-NMR** (CDCl₃, 300 MHz): δ= 7,65 (d, 1H, *³J* = 8,3 Hz), 6,8 (d, 1H, *³J* = 8,3 Hz), 5,03 (d, 1H, *³J* = 8,3 Hz), 4,55-4,45 (m, 1H), 3,99-3,89 (m, 2H), 3,57 (dd, 1H, *³J* = 3,3 Hz, *²J* = 11,0 Hz), 3,47 (dd, 1H, *³J* = 3,3 Hz, *²J =* 11,0 Hz), 2,99 (d, 1H, *³J*= 4,3 Hz), 2,58 (d, 1H, *³J =* 4,3 Hz), 2,48 (d, 1H, *³J*= 4,3 Hz), 2,15-2,14 (m, 2H), 1,44 ( s, 9H), 1,34 (s, 9H), 0,90-0,87 (m, 6H), 0,80-0,75 (m, 6H) ppm.
**¹³C-NMR** (CDCl₃, 75 MHz): δ= 173,0, 171,5, 170,5, 156,3, 81,9, 81,0, 65,6, 54,3, 50,6, 50,5, 40,8, 39,7, 35,9, 28,4, 28,0, 24,9, 23,2, 22,2, 21,6, 21,1 ppm.
MS (EI): *m*/*z* = 501 (M⁺).

### C) Verbindung 13 (BSc2115).

Verbindung 7 (BSc2114) (400mg, 0,8 mmol) wurde mit IBX (2-Iodoxybenzoesäure, 268 mg, 0,95 mmol) in DMSO (5 mL) für 6 h bei Raumtemperatur oxidiert. CH₂Cl₂ (30 mL) wurde zugefügt und die Lösung mit Wasser (3 x 30mL), NaHCO₃-Lösung (3 x 30 mL, gesättigt), gesättigter NaCl-Lösung (1 x 30 mL) gewaschen. Das Lösungsmittel wurde nach dem Trocknen (Na₂SO₄) unter Vakuum entfernt, um Verbindung **13** (BSc2115) zu erhalten (390 mg, 98%).
**¹H-NMR** (CDCl₃, 300 MHz): δ= 9.4 (s, 1H), 7,54 (d, 1H, *³J* = 8,3 Hz), 7,27 (d, 1H, *³J* = 8,3 Hz), 4,89 (d, 1H, *³J*= 8,3 Hz), 4,66-4,56 (m, 2H), 4,22-4,15 (m, 2H), 3,99 (dd, 1H, *³J*= 3,3 Hz, *²J=* 11,0 Hz), 2,99 (dd, 1H, *³J =* 3,3 Hz, *²J=* 11,0 Hz), 2,90 (d, 1H, *³J*= 4,3 Hz), 2,58 (d, 1H, *³J* = 4,3 Hz), 2,48 (d, 1H, *³J*= 4,3 Hz), 1,66-1,55 (m, 1H), 1,47-1,45 (m, 1H), 1,44 (s, 9H), 1,34 (s, 9H), 0,9-0,86 (m, 6H,), 0,80-0,76 (m, 6H) ppm.
**¹³C-NMR** (CDCl₃, 75 MHz): δ = 200,1, 172,6, 171,6, 170,9, 156,2, 82,0, 80,8, 54,3, 50,6, 50,5, 40,8, 39,7, 35,9, 28,4, 28,0, 24,9, 23,2, 22,2, 21,6, 21,1 ppm.
**MS** (EI): *m*/*z* = 499 (M⁺).

### D) Verbindung 14 (BSc2128).

TFA (1 mL) wurde zu einer gerührten Lösung von Verbindung **7** (BSc2114. 390 mg, 0,78 mmol) in CH₂Cl₂ (4 mL) zugefügt. Das Lösungsmittel wurde nach 3h verdampft, um Verbindung **14** (BSc2128) zu erhalten (260 mg, 97%).
**¹H-NMR** (DMSO-d₆, 300 MHz): δ= 9,37 (s, 1H), 8,85 (d, 1H, *³J*= 8,3 Hz), 8,19 (d, 1H, *³J*= 8,3 Hz), 4,67-4,56 (m, 1H), 4,05-3,89 (m, 2H), 3,77-3,65 (m, 2H), 3,70-3,67 (m, 1H), 2,89 (dd, 1H, *³J* = 4,1 Hz, *²J =* 16,0 Hz), 2,78 (d, 1H, *³J* = 4,1 Hz, *²J =* 16,0 Hz), 2,58 (d, 1H, *³J* = 4,1 Hz), 2,48 (d, 1H, ,*³J*= 4,1 Hz), 1,60-1,58 (m, 1H), 1,46-1,44 (m, 1H), 0,80-0,76 (m, 6H), 0,70-0,66 (m, 6H) ppm.
**¹³C-NMR** (DMSO-d₆, 75 MHz): δ = 200,1, 171,1, 171,0, 169,9, 55,2, 53,8, 50,4, 40,8, 39,7, 35,9, 23,2, 22,2, 21,6, 21,1 ppm.
**MS** (ESI): *m*/*z* = 343.4 (M⁺).

### E) Die Verbindungen 16 (BSc2129), 9 (BSc2207), 17 (BSc2208), 18 (BSc2197) und 12 (BSc2194) wurden durch analoge Verfahren dargestellt.

### F) Verbindung 8 (BSc2117).

Die Verbindung 8 wurde aus Verbindung **33** gemäß der gleichen Prozedur dargestellt und mit 80% Ausbeute erhalten.
¹H-NMR (CDCl₃, 300 MHz): δ=7,51 (d, 1H, *³J* = 8,3 Hz),7,29-7,19 (m, 5H), 6,70 (d, 1H, *³J* = 8,3 Hz), 5,35 (d, 1H, *³J* = 8,3 Hz), 5,50 (s, 2H), 4,60-4,58 (m, 1H), 4,10-4,75 (m, 1H), 3,95-3,87 (m, 1H), 3,58 (dd, 1H, *³J* = 3,3 Hz, *²J=* 11,0 Hz), 3,45 (dd, 1H, *³J=* 3,3 Hz, *²J* = 11,0 Hz), 2,89 (dd, 1H, *³J=* 4,1 Hz, *²J=* 16,0 Hz), 2,80 (d, 1H, *³J* = 4,1 Hz, *²J* = 16,0 Hz), 2,59 (d, 1H, *³J* = 4,3 Hz,), 2,50 (d, 1H, *³J=* 4,3 Hz,), 2,0-1,96 (m, 2H), 1,55-1,53 (m, 1H), 1,34 (s, 9H), 1,25-1,23 (m, 1H), 0,89 (dd, 6H, *³J* = 4,3 Hz, *³J* = 7,0 Hz), 0,80 (dd, 6H, *³J* = 4,3 Hz, *³J* = 7,0 Hz) ppm.
**¹³C-NMR** (CDCl₃, 75 MHz): δ=167,0, 166,2, 165,1, 151,4, 130,4, 123,3, 123,1, 123,1, 122,8, 77,1, 62,2, 60,2, 49,0, 45,8, 45,2, 35,6, 34,4, 31,1, 22,7, 22,7, 22,7, 21,6, 21,1, 19,5, 17,8 ppm. **MS** (EI): *m*/*z* = 535(M⁺).
**G) Verbindung 15 (BSc2118).**

Die Verbindung **15** wurde aus Verbindung **8** (BSc2117) gemäß der gleichen Prozedur dargestellt und mit 94% Ausbeute erhalten.
**¹H-NMR** (CDCl₃, 300 MHz): δ= 9,49 (s, 1H), 7,48 (d, 1H, *³J*= 8,3 Hz), 7,35-7,33 (m, 5H), 7,25 (d, 1H, *³J*= 7,3 Hz), 5,23-5,22 (m, 1H), 5,12 (s, 2H), 4,80-4,79 (m, 1H), 4,38-4,37 (m, 1H), 4,15-4,14 (m, 1H), 3,00 (d, 1H, *³J* = 3,3 Hz), 2,98 (d, 1H, *³J* = 3,3 Hz), 2,60 (d, 1H, *³J* = 6,3 Hz), 2,55 (d, 1H, *³J* = 6,3 Hz), 2,30-2,28 (m, 1H), 2,23-2,22 (m, 1H), 2,05-1,99 (m, 2H), 1,77-1,76 (m, 1H), 1,44 (s, 9H), 1,35-1,34 (m, 1H), 0,89-0,86 (m, 6H), 0,80-0,78 (m, 6H) ppm. **¹³C-NMR** (CDCl₃, 75 MHz): δ=200, 172,1, 171,6, 170,8, 67,4, 156,6 , 135,9, 128,7, 128,4, 128,1, 122,8, 82,1, 57,5, 54,5, 49,8, 45,8, 45,2, 41,1, 37,4, 36,5, 28,0, 28,0, 28,0, 24,5, 23,3, 23,0, 21,7 ppm.
MS (ESI): *mlz* = 533 (M⁺).

### H) Verbindung 16 (BSc2129).

Die Verbindung **16** wurde aus Verbindung **15** (BSc2118) gemäß der gleichen Prozedur dargestellt und mit 84% Ausbeute erhalten.
**¹H-NMR** (DMSO-d₆, 300 MHz): δ= 9,8 (s, 1H), 9,37 (s, 1H), 8,30 (d, 1H, *³J*= 8,3 Hz), 8,24 (d, 1H, *³J* = 7,3 Hz), 7,36-7,34 (m, 5H), 5,22-5,21 (m, 1H), 5,12 (s, 2H), 4,80-4,79 (m, 1H), 4,5-4,45 (m, 1H), 4,15-4,10 (m, 1H), 3,30-3,29 (m, 1H), 2,98-2,97 (m, 1H), 2,60-2,59 (m, 1H), 2,55-2,54 (m, 1H), 2,30-2,29 (m, 1H), 2,23-2,22 (m, 1H), 2,05-1,99 (m, 2H), 1,77-1,76 (m, 1H), 1,35-1,34 (m, 1H), 0,89-0,88 (m, 6H), 0,80-0,79 (m, 6H) ppm.
**¹³C-NMR** (DMSO-d₆, 75 MHz): δ=200, 172,1, 171,6, 170,8, 67,4, 156,6, 135,9, 128,7, 128,4, 128,1, 122,8, 82,1, 57,5, 54,5, 49,8, 45,8, 45,2, 41,1, 37,4, 36,5, 24,5, 23,3, 23,0 , 21,7 ppm. MS (ESI): *m*/*z* = 476 (M⁺).

### I) Verbindung 9 (BSc2207).

Die Verbindung **9** wurde aus Verbindung **33** gemäß der gleichen Prozedur dargestellt und mit 93% Ausbeute erhalten.
**¹H-NMR** (CDCl₃, 300 MHz): δ=7,67 (d, 1H, *³J* = 8,3 Hz), 7,4 (d, 1H, *³J=* 9,2 Hz), 7,26 (d, 1H, *³J =* 8,4 Hz), 4,7-4,63 (m, 2H), 4,43-4,33 (m, 2H), 4,04-3,94 (m, 2H), 3,59-3,54 (m, 1H), 3,53-3,43 (m, 1H), 3,30 (dd, 1H, *³J* = 4,8 Hz, *²J* = 17,0 Hz), 2,8 (dd, 1H, *³J* = 4,8 Hz, *²J* = 17,0 Hz), 2,65-2,55 (m, 1H), 2,54-2,45 (m, 1H), 2,15-2,05 (m, 2H), 2,0 (d, 3H, *³J* = 15,0 Hz), 1,44 (s, 9H), 0,9-0,87 (m, 6H), 0,80-0,78 (m, 6H) ppm.
**¹³C-NMR** (CDCl₃, 75 MHz): δ= 172,5, 172,3, 171,6, 169,9, 82,0, 66,6, 53,4, 50,4, 49,5, 40,8, 39,7, 35,9, 28,4, 25,0, 24,7, 22,9, 23,2, 22,1 ppm.
**MS** (EI): *m*/*z* = 443 (M⁺).

### J) Verbindung 10 (BSc2195).

Die Verbindung **10** wurde aus Verbindung **34** gemäß der gleichen Prozedur dargestellt und mit 88% Ausbeute erhalten.
**¹H-NMR** (CDCl₃, 300 MHz): δ= 8,26 (d, 1H, *³J* = 7,2 Hz), 7,59 (m, 5H, ArH), 6,70 (d, 1H, *³J* = 7,3 Hz), 6,35 (d, 1H, *³J* = 7,0 Hz), 5,20 (s, 2H), 4,62-4,48 (m, 1H), 4,44-4,42 (m, 1H), 4,01-4,00 (m, 1H), 3,98-3,97 (m, 1H), 3,91-3,90 (m, 1H), 2,89-2,88 (dm, 1H), 2,80-2,78 (m, 1H), 2,59-2,58 (m, 1H), 2,50-2,49 (m, 1H), 2,0-1,99 (m, 2H), 1,55-1,54 (m, 1H), 1,34 (s, 3H), 1,25-1,24 (m, 1H), 0,89 (dd, 6H, *³J=* 3,8 Hz, 6,7 Hz), 0,80 (dd, 6H, *³J=* 3,8 Hz, 6,7 Hz) ppm.
**¹³C-NMR** (CDCl₃, 75 MHz): δ= 175,5, 173,7, 173,0, 169,7, 128,8, 128,3, 68,5, 66,5, 41,4, 40,8, 40,2, 35,6, 34,4, 31,1, 23,5, 22,1, 23,9, 23,6, 22,9 ppm.
**MS** (EI): *m*/*z* = 477 (M⁺).

### K) Verbindung 17 (BSc2208).

Die Verbindung **17** wurde aus Verbindung **10** (BSc2195) gemäß der gleichen Prozedur dargestellt und mit 74% Ausbeute erhalten..
**¹H-NMR** (CDCl₃, 300 MHz): δ= 9,40 (s, 1H), 8,26 (d, 1H, *³J* = 7,0 Hz), 7,28-7,19 (m, 5H), 6,70 (d, 1H, *³J* = 7,0 Hz), 5,65 (d, 1H, *³J* = 7,0 Hz), 5,30 (s, 2H), 4,82-4,81 (m, 1H), 4,35-4,33 (m, 1H), 3,96-3,95 (m, 1H), 2,80-2,79 (m, 1H), 2,75-2,74 (m, 1H), 2,49-2,48 (m, 1H), 2,45-2,44 (m, 1H), 2,0-1,98 (m, 2H), 1,51-1,50 (m, 1H), 1,30 (s, 3H), 1,25-1,24 (m, 1H), 0,89 (dd, 6H, *³J* = 3,8 Hz, 6,4 Hz), 0,80 (dd, 6H, *³J* = 3,8 Hz, 6,4 Hz) ppm.
**¹³C-NMR** (CDCl₃, 75 MHz): δ = 200, 174,5, 173,5, 173,0, 166,7, 128,6, 128,3, 68,5, 41,4, 40,8, 40,2, 35,6, 34,4, 31,1, 23,5, 22,1, 23,9, 23,6, 22,9.
**MS** (EI): *m*/*z* = 475 (M⁺).

### L) Verbindung 11 (BSc2196).

Die Verbindung **11** wurde aus Verbindung **34** gemäß der gleichen Prozedur dargestellt und mit 77% Ausbeute erhalten.
**¹H-NMR** (CDCl₃, 300 MHz): δ= 7,29-7,19 (m, 10H), 6,65 (d, 1H, *³J* = 7,0 Hz), 6,33 (d, 1H, *³J* = 7,0 Hz), 5,35 (d, 1H, *³J* = 7,0 Hz), 5,28-5,17 (m, 4H), 4,22-4,21 (m, 1H), 4,0-3,99 (m, 1H), 3,74-3,73 (m, 1H), 3,66-3,65 (m, 1H), 3,22-3,21 (m, 1H), 2,99-2,98 (m, 1H), 2,92-2,91 (m, 1H), 2,62-2,60 (d, 1H), 2,50-2,51 (d, 1H,), 2,4-2,3 (m, 2H), 1,57-1,56 (m, 1H), 1,24-1,23 (m, 1H), 0,87 (dd, 6H, *³J* = 3,8 Hz, 6,4 Hz), 0,80 (dd, 6H, *³J=* 3,8 Hz, 6,4 Hz) ppm.
**¹³C-NMR** (CDCl₃, 75 MHz): δ= 172,4, 172,0, 170,2, 156,8, 142,8, 135,8, 128,7, 68,5, 67,2, 62,2, 52,0, 49,8, 49,2, 41,6, 40,4, 33,1, 22,4, 22,1, 21,6, 21,1, 19,5, 18,3 ppm.
**MS** (EI): *m*/*z* = 569 (M⁺).

### M) Verbindung 18 (BSc2197).

Die Verbindung **18** wurde aus Verbindung **11** (BSc2196) gemäß der gleichen Prozedur dargestellt und mit 74% Ausbeute erhalten..
**¹H-NMR** (CDCl₃, 300 MHz): δ= 9,33 (s, 1H), 7,26-7,19 (m, 10H), 7,03 (d, 1H, *³J*= 7,0 Hz), 6,41 (d, 1H, *³J* = 7,0 Hz), 5,66 (d, 1H, *³J* = 7,0 Hz), 5,23-4,95 (m, 4H), 4,26-4,13 (m, 1H), 4,12-4,03 (m, 1H), 3,57-3,54 (m, 1H), 2,94 (d, 1H, *³J =* 7,3 Hz), 2,72 (d, 1H, *³J =* 7,3 Hz), 2,02 (d, 1H, *³J* = 10,0 Hz), 1,99 (d, 1H, *³J* = 10,0 Hz), 1,57-1,44 (m, 2H), 1,45-1,42 (m, 1H), 1,24-1,99 (m, 1H), 0,87 (dd, 6H, *³J* = 3,8 Hz, 6,4 Hz ), 0,80 (dd, 6H, *³J* = 3,8 Hz, 6,4 Hz) ppm.
**¹³C-NMR** (CDCl₃, 75 MHz): δ= 200, 173,4, 172,6, 171,2, 156,8, 142,8, 138,8, 128,7, 128,7, 66,5, 65,2, 52,0, 49,7, 48,2, 41,7, 40,6, 33,5, 22,6, 22,3, 21,9, 21,5, 19,4, 18,0 ppm.
**MS** (ESI): *m*/*z* = 567 (M⁺).

### N) Verbindung 12 (BSc2194).

Die Verbindung **12** wurde aus Verbindung **34** gemäß der gleichen Prozedur dargestellt und mit 90% Ausbeute erhalten.
**¹H-NMR** (CDCl₃, 300 MHz): δ= 7,32-7,28 (m, 5H), 7,01 (d, 1H, *³J=* 7,0 Hz), 6,33 (d, 1H, *³J* = 7,0 Hz), 5,32 (s, 2H), 4,91 (d, 1H, *³J* = 7,0 Hz), 4,25-4,22 (m, 1H), 4,08-4,06 (m, 1H), 3,98-3,97 (m, 1H), 3,68-3,65 (dd, 2H), 2,99-2,97 (m, 1H), 2,92 (d, 1H, *³J* = 7,3 Hz), 2,59 (d, 1H, *³J* = 7,3 Hz), 2,50 (d, 1H, *³J* = 7,3 Hz), 1,97-1,87 (m, 2H), 1,55-1,54 (m, 1H), 1,34 (s, 9H), 1,24,1,23 (m, 1H), 0,89 (dd, 6H, *³J* = 3,8 Hz, 6,4 Hz), 0,80 (dd, 6H, *³J* = 3,8 Hz, 6,4 Hz) ppm. **¹³C-NMR** (CDCl₃, 75 MHz): δ= 172,8, 172,4, 170,1, 155,8, 135,4, 128,6, 77,1 , 76,6, 67,2, 62,2, 49,0, 45,8, 45,2, 35,6, 34,4, 33,1, 23,7, 22,4, 22,1, 21,6, 21,1, 19,5, 18,3 ppm.
**MS** (EI): *m*/*z* = 535 (M⁺).

### O) Verbindung 19 (BSc2158) (Referenzbeispiel)

Eine Suspension von NaH in Mineralöl (60%, 291 mg, 7,3 mmol) in THF (2 mL, abs.) wurde mit (*S*)-(-)-Phenylglycinol (500 mg, 3,6 mmol) bei -15°C unter Argonatmosphäre behandelt und für 20 min gerührt. Die Mischung wurde auf -55°C gekühlt, ehe Trichloroethylen (400 µl, 4.5 mmol) in THF (2 mL) zugefügt wurde. Die Mischung wurde innerhalb 5 h auf Umgebungstemperatur erwärmt, mit Wasser (40 mL) versetzt und anschließend mit Et₂O (60 mL) extrahiert. Die organische Schicht wurde extrahiert, mit gesättigter NaCl-Lösung (40 mL) gewaschen, getrocknet (Na₂SO₄) und konzentriert. Das Produkt wurde durch Säulenchromatographie gereinigt, um den Dichlorvinylether **35** zu ergeben (347 mg, 56%). Zu einer Mischung von Z-Leu-Leu-OH (378 mg, 1,0 mmol), EDAC (192 mg, 1,0 mmol) und HOBt (170 mg, 1.1 mmol) wurde DMF (2 mL) zugefügt. Die resultierende Lösung wurde für 10 min kräftig gerührt, danach wurden der Dichlorvinylether **35** (200 mg, 1,2 mmol) und Et₃N (0,28 mL, 2,0 mmol) addiert. Die Lösung wurde für 2 h gerührt. DCM (40 mL) wurde addiert und es wurde mit Salzsäure (0,1 N, 3 x 30 mL), wäßrigem NaHCO₃ (gesättigt, 3 x 30 mL) und Wasser (3 x 30 mL) gewaschen. Die organische Phase wurde getrocknet (Na₂SO₄) und das Lösungsmittel unter Vakuum entfernt, um Verbindung **19** (BSc2158) zu erhalten (403 mg, 77%).
**¹H-NMR** (DMSO-d₆, 300 MHz): δ= 8,52 (d, 1H, *³J* = 8,3 Hz), 8,33 (d, 1H, *³J* = 8,3 Hz), 7,44 (d, 1H, *³J* = 8,2 Hz, NH-Leu1), 7,40-7,28 (m, 10H), 6,07 (s, 1H), 5,19-5,12 (m, 1H), 5,00 (s, 2H), 4,45-4,40 (m, 1H), 4,22-4,11 (m, 2H), 4,08-4,01 (m, 1H), 1,65-1,35 (m, 6H), 0,90-0,78 (m, 12H) ppm.
**¹³C-NMR** (DMSO-d₆, 75 MHz): δ= 172,0, 171,5, 155,8, 142,2, 138,4, 136,9, 128,2, 127,7, 127,6, 127,4, 127,5, 126,9, 97,7, 73,0, 65,2, 53,0, 51,4, 50,8, 40,8, 40,6, 24,1, 24,0, 22,6, 21,9, 21,6, 21,3 ppm.
**MS** (EI): *m*/*z =* 480 (Z-Leu-Leu-C₈H₉⁺), 371 (CO-Leu-C₁₀H₁₀Cl₂NO⁺).

**P) Verbindung 20 (BSc2166)** (Referenzbeispiel).

Der Dichlorvinylether **36** (178 mg, 25%) wurde von (*S*)-(-)-Leucinol (400 mg, 3,4 mmol) gemäß der Synthese des Dichlorvinylethers **35** synthetisiert. Die Reaktion wurde bei -70°C gestartet, über Nacht auf Raumtemperatur erwärmt, danach für weitere 60 h gerührt. Die Kupplung von Verbindung **36** (100 mg, 0,47 mmol) mit Z-Leu-Leu-OH (100 mg, 0.47 mmol) ergab Produkt **20** (BSc2166) (143 mg, 63%).
**¹H-NMR** (DMSO-d₆, 300 MHz): δ= 7,92 (d, 1H, *³J=* 5,1 Hz), 7,70 (d, 1H, *³J*= 5,1 Hz), 7,48 (d, 1H, *³J* = 4,2 Hz), 7,40-7,31 (m, 5H), 6,05 (s, 1H), 5,05 (s, 2H), 4,33-4,29 (m, 1H), 4,08-4,03 (m, 2H), 3,87-3,85 (m, 2H), 1,60-1,41 (m, 9H,) 0,90-0,88 (m, 18H) ppm.
**¹³C-NMR** (DMSO-d₆, 75 MHz): δ = 171,9, 171,5, 155,8, 142,9, 137,0 128,2, 127,7, 127,5, 97,9, 73,3, 65,3, 53,1, 50,9, 45,7, 40,9, 40,0, 39,8, 24,11, 24,08, 23,85, 23,16, 22,92, 22,87, 21,80, 21,50, 21,5 ppm.
**MS** (EI): *m*/*z* = 460 (Z-Leu-Leu-C₆H₁₄N⁺), 295 (CO-Leu-C₄H₆Cl₂NO⁺).

### Q) Verbindung 21 (BSc2167) (Referenzbeispiel).

Ein im Ofen getrockneter Rundkolben wurde mit Zn(OTf)₂ (168 mg, 0,45 mmol) und (-)-Ephedrin (84 mg, 0,50 mmol) unter Argonatmosphäre beladen. Et₃N (51 mg, 70 µL, 0,50 mmol) in trockenem Toluol (2 mL) wurde addiert und für 2 h bei Raumtemperatur gerührt. Verbindung **3** (MG132) (200 mg, 0,42 mmol) und Phenylacetylen (52 mg, 56 µL, 0,50 mmol) wurden nach weiteren 15 min addiert und für 20 h bei 60°C gerührt. CH₂Cl₂ (40 mL) und ein wäßriger KH₂PO₄/Na₂HPO₄-Puffer (30 mL, pH 5,5) wurden addiert, die organische Schicht wurde abgetrennt und die wäßrige Phase mit CH₂Cl₂ (2 x 30 mL) extrahiert. Die vereinigten organischen Schichten wurden getrocknet (Na₂SO₄) und das Lösungsmittel im Vakuum entfernt. Das Rohprodukt wurde mittels Säulenchromatographie gereinigt, um Verbindung **21** (BSc2167) zu ergeben (260 mg, 54%).
**¹H-NMR** (DMSO-d₆, 300 MHz): δ = 8,46-8,37 (m, 1H), 7,50-7,47 (m, 1H), 7,43-7,19 (m, 10H), 5,20-5,11 (m, 1H), 5,00-4,95 (m, 2H), 4,57-4,49 (m, 2H), 4,13-4,03 (m, 2H), 1,80-1,24 (m, 9H), 0,90-0,53 (m, 18H) ppm.
**¹³C-NMR** (DMSO-d₆, 75 MHz): δ = 174,2, 174,7, 156,9, 139,5, 131,2, 127,9, 127,8, 127,6, 127,3, 126,5, 126,3, 98,1, 80,0, 63,1, 62,8, 54,5, 52,9, 46,8, 40,9, 40,3, 36,9, 24,7, 24,2, 24,0, 23,2, 23,1, 22,9, 21,4, 21,2, 20,7 ppm.
**MS** (EI) *m*/*z* = 446 (Z-Leu-Leu-C₅H₁₁⁺), 257 (CO-Leu-C₆H₁₃O⁺).

### R) Verbindung 22 (BSc2160) (Referenzbeispiel).

Z-Leu-Leu-OH (151 mg, 0,4 mmol) und (*S*)-3-Amino-1-chlor-5-methylhexan-2-on (80 mg, 0,4 mmol) wurden, wie für Verbindung **19** (BSc2158) beschrieben, gekoppelt. Das Rohprodukt wurde mittels Säulenchromatographie gereinigt, um Verbindung **22** (BSc2160) zu ergeben (32 mg, 16%).
**¹H-NMR** (CDCl₃, 300 MHz): δ= 7,35-7,19 (m, 5H), 6,81-6,73 (m, 1H), 6,41-6,32 (m, 1H), 5,16 (d, 1H, *³J* = 5,1 Hz), 5,03 (s, 2H), 4,64-4,61 (m, 1H), 4,35-4,06 (m, 3H), 1,87-1,40 (m, 9H), 0,92-0,73 (m, 18H) ppm.
**¹³C-NMR** (CDCl₃, 75 MHz): δ= 201,4, 172,7, 172,3, 156,6, 136,2, 128,6, 128,3, 128,0, 67,2, 55,0, 53,8, 51,8, 47,1, 41,4, 40,6, 39,6, 24,9, 24,8, 23,3, 22,9, 22,7, 22,6, 22,3, 22,2, 21,5 ppm. **MS** (EI): *m*/*z* = 488 (Z-Leu-Leu-Leu-CH₂⁺), 432 (OCO-Leu-Leu-Leu-CH₂Cl⁺).

### S) Verbindung 23 (BSc2159) (Referenzbeispiel).

Verbindung **3** (MG132) (300 mg, 0,63 mmol), Benzylisonitril (116 µl, 0,95 mmol), und Pyridin (204 µl, 2,53 mmol) wurden in CH₂Cl₂ (2,0 mL) gelöst und auf -10°C gekühlt. Trifluoressigsäure (97 µL, 1,26 mmol) wurde tropfenweise unter Argonatmosphäre über 15 min addiert (T < 0°C). Die Kühlung wurde für 2 h fortgesetzt, wonach zusätzliche 72 h bei Raumtemperatur folgten. CH₂Cl₂ (50 mL) wurde addiert und es wurde mit Salzsäure (0,1 N, 3 x 30 ml), wäßrigem NaHCO₃ (gesättigt, 3 x 30 mL) und gesättigter NaCl-Lösung (3 x 40 ml) gewaschen. Die organische Phase wurde getrocknet (Na₂SO₄) und mittels Säulenchromatographie gereinigt, um Verbindung **23** (BSc2159) zu ergeben (191 mg, 50%). **¹H-NMR** (CDCl₃, 300 MHz): δ = 7,51-7,27 (s, 1H), 7,26-7,14 (m, 10H), 6,91-6,80 (m, 1H) 5,82-5,63 (m, 2H), 5,02-4,90 (m, 2H), 4,35 (d, 1H, *³J* = 10,7 Hz), 4,30-4,01 (m, 5H), 1,55-1,31 (m, 9H), 0,81-0,73 (m, 18H) ppm.
**¹³C-NMR** (CDCl_{3,} 75 MHz): δ = 173,3, 172,7, 172,6, 157,5, 138,0, 136,2, 128,9, 128,8, 128,6, 128,1, 127,9, 127,8, 73,5, 67,3, 53,9, 52,0, 51,7, 43,2, 42,4, 41,6, 25,0, 24,8, 23,3, 23,0, 22,1, 22,0, 21,6 ppm.
**MS** (EI): *m*/*z* = 610 (M⁺).

### T) Verbindung 24 (BSc2185) (Referenzbeispiel).

Verbindung **3** MG132 (145 mg, 0,3 mmol) und 3-Picolylisonitril (52 mg, 0,45 mmol) wurden gemäß der Präparation von Verbindung **23** (BSc2159) in das α-Hydroxylamid **24** (BSc2185) umgewandelt. Reinigung mittels Säulenchromatographie ergab Verbindung **24** (BSc2185) (103 mg, 56%).
**¹HNMR** (CDCl₃, 300 MHz): δ = 8,62-8,36 (m, 2H), 7,71-7,50 (m, 2H), 7,28-7,20 (m, 6H), 7,05-6,96 (m, 1H) 5,92-5,57 (m, 2H), 5,00 (s, 2H), 4,72-4,56 (m, 1H), 4,46-4,10 (m, 5H), 1,79 (s, 1H), 1,62-1,18 (m, 9H), 0,92-0,73 (m, 18H) ppm.
**¹³C-NMR** (DMSO-d_{6,} 75 MHz): δ = 172,4, 171,9, 171,2, 155,8, 148,9, 147,9, 137,0, 135,2, 135,0, 128,2, 127,7, 127,6, 123,2, 73,8, 65,2, 53,0, 51,0, 49,2, 42,8, 40,6, 37,1, 24,1, 23,7, 22,2, 23,0, 21,8, 21,7, 21,6, 21,1 ppm.
**MS** (EI) *m*/*z* = 611 (M⁺).

### U) Verbindung 25 (BSc2186) (Referenzbeispiel).

Verbindung **3** MG132 (200 mg, 0,42 mmol) und Phenylisonitril (65 mg, 0,63 mmol) wurden gemäß der Präparation von Verbindung 23 (BSc2159) in das α-Hydroxylamid **25** (BSc2186) umgewandelt. Das Rohprodukt wurde mittels Säulenchromatographie gereinigt, um Verbindung **25** (BSc2186) zu ergeben (70 mg, 28%).
**¹H-NMR** (DMSO-d₆, 300 MHz): δ = 8,27 (s, 1H), 7,72-7,57 (m, 2H), 7,37-7,20 (m, 7H), 7,11-7,01 (m, 2H), 6,11-6,08 (m, 1H), 5,95-5,92 (m, 1H), 5,07 (s, 2H), 4,30-4,24 (m, 2H), 4,07-3,99 (m, 2H), 1,57-1,32 (m, 9H), 0,88-0,61 (m, 18H) ppm.
**¹³C-NMR** (DMSO-d_{6,} 75 MHz): δ = 172,0, 171,1, 171,0, 155,7, 138,3, 137,0, 128,4, 128,2, 127,6, 127,5, 123,5, 119,5, 65,2, 52,7, 52,9, 49,2, 40,5, 40,3, 40,0, 24,1, 23,9, 23,8, 23,1, 23,0, 22,6, 22,0, 21,3, 21,2 ppm.
**MS** (EI) *m*/*z* = 596 (M⁺).

### V) Verbindung 26 (BSc2187) (Referenzbeispiel).

α-Hydroxylamid **23** (BSc2159) (40 mg, 0,065 mmol) und IBX (36 mg, 0,13 mmol) wurden in DMSO gelöst und bei Raumtemperatur für 12 h gerührt. DCM (40 mL) und Wasser (30 mL) wurden vor der Filtration addiert. Die organische Schicht wurde abgetrennt und mit Wasser (2 x 40 mL), wäßrigem NaHCO₃ (1 x 40 mL, 0,05 N) und Wasser (1 x 30 mL) gewaschen. Die organische Schicht wurde getrocknet (Na₂SO₄) und das Lösungsmittel im Vakuum entfernt, um Verbindung **26** (BSc 2187) zu ergeben (22 mg, 56%).
**¹H-NMR** (CDCl₃, 300 MHz): δ = 7,50-7,41 (m, 1H), 7,41-7,14 (m, 10H), 6,91-6,80 (m, 1H) 5,58-5,54 (m, 1H), 5,27-5,21 (m, 1H), 5,03-4,94 (m, 2H), 4,43-4,31 (m, 3H), 4,19-4,03 (m, 2H), 1,64-1,12 (m, 9H), 0,92-0,74 (m, 18H) ppm.
**¹³C-NMR** (CDCl_{3,} 75 MHz): δ = 191,8, 172,6, 171,8, 161,4, 156,4, 136,9, 136,3, 128,9, 128,9, 128,6, 128,3, 128,1, 128,0, 67,2, 53,6, 53,3, 51,6, 43,4, 41,5, 40,86, 40,0, 25,3, 24,8, 23,8, 23,7, 23,3, 23,0, 22,8, 22,4, 22,1, 21,5 ppm.
MS (EI) *m*/*z* = 474 (Z-Leu-Leu-C₆H₁₂NO⁺).

### W) Verbindung 27 (BSc2188) (Referenzbeispiel).

Verbindung **24** (BSc2185) wurde gemäß der Synthese von Verbindung **26** (BSc2187) oxidiert. Reinigung mittels Säulenchromatographie ergab Verbindung **27** (BSc2188) (60 mg, 49%).
**¹H-NMR** (CDCl₃, 300 MHz): δ = 8,46-8,44 (m, 2H), 7,64-7,47 (m, 2H), 7,24-7,12 (m, 6H), 6,85-6,82 (m, 1H), 5,54-5,47 (m, 1H), 5,22-5,15 (m, 1H), 5,04-4,93 (m, 2H), 4,46-3,99 (m, 4H), 1,62-1,16 (m, 9H), 0,92-0,76 (m, 18H) ppm.
**¹³C-NMR** (CDCl_{3,} 75 MHz): δ = 196,4, 172,6, 171,9, 159,8, 156,4, 149,3, 149,2, 136,2, 135,9, 133,0, 128,7, 128,3, 128,0, 123,8, 67,2, 53,7, 53,3, 51,5, 41,4, 40,8, 40,6, 39,9, 25,0, 24,8, 23,3, 23,0, 22,1, 22,0 ppm.
**MS** (EI): *m*/*z* = 609 (M⁺), 474 (Z-Leu-Leu-C₆H₁₂NO⁺).

### X) Verbindung 28 (BSc2189) (Referenzbeispiel).

Verbindung **25** (BSc2185) (200 mg, 0,35 mmol) wurde gemäß der Synthese von Verbindung **26** (BSc2187) oxidiert. Reinigung mittels Säulenchromatographie ergab Verbindung **28** (BSc2189) (30 mg, 50%).
**¹H-NMR** (CDCl₃, 300 MHz): δ = 8,58 (s, 1H), 7,56-7,54 (m, 2H), 7,29-7,19 (m, 7H), 7,11-7,00 (m, 1H), 6,75 (d, 1H, *³J* = 9,0 Hz), 6,63 (d, 1H, *³J* = 9,1 Hz), 5,36-5,28 (m, 2H), 5,07 (s, 2H), 4,51-4,41 (m, 1H), 4,19-4,11 (m, 1H), 1,97-1,41 (m, 9H), 0,93-0,77 (m, 18H) ppm.
**¹³C-NMR** (CDCl₃, 75 MHz): δ = 196,8, 172,5, 171,8, 157,2, 156,4, 136,3, 136,2, 129,3, 129,2, 128,7, 125,5, 120,0, 67,3, 53,7, 53,0, 51,6, 41,3, 40,6, 40,9, 25,4, 25,4, 24,8, 23,3, 23,0, 22,8, 22,2, 22,1, 21,5 ppm.
**MS** (EI): *m*/*z* = 474 (Z-Leu-Leu-C₆H₁₂NO⁺).

### Beispiel 2: Isolation von Proteasomen

Proteasomen wurden aus roten Blutzellen isoliert. Zellen wurden mit DTT (1 mM) lysiert und der stromafreie Überstand wurde auf DEAE-Sepharose (Toyopearls) aufgetragen. Proteasom wurde mit einem NaCl-Gradient in TEAD (20 mM TrisCl, pH 7,4, 1 mM EDTA, 1 mM Azid, 1mM DTT) von 100 bis 300 mM NaCl eluiert. Proteasom wurde durch AmmoniumsulfatPräzipitation (zwischen 40 und 70% Sättigung) konzentriert und in einem 10-40% Sacharose-Gradient durch Zentrifugation bei 40.000 rpm für 16 Stunden separiert (SW40; L7, Beckman & Coulter). Letztendlich wurde Proteasom an einer MonoQ-Säule gereinigt und mit einem NaCl-Gradienten bei ca. 280 mM NaCl eluiert. Die Fraktionen, die gereinigtes Proteasom enthielten, wurden gegen 50 mM NaCl in TEAD dialysiert und auf Eis gelagert. Die Reinheit wurde durch SDS-PAGE bestimmt.

### Beispiel 3: Protease-Assays

Suc-LLVY-AMC, Z-VGR-AMC und LLE-AMC (BACHEM, Calbiochem) wurden verwendet, um die Chymotrypsin-artigen, Trypsin-artigen bzw. Caspase-artigen (postsauren)-Aktivitäten des Proteasoms zu bestimmen. Substrate wurden mit Proteasom bei 37°C in Assay-Puffer (20 mM Tris/Cl, pH 7,2, 1 mM EDTA, 1 mM DTT) für eine Stunde inkubiert. 100 ng Proteasom wurde mit 0,01 -10 µM der Inhibitoren für 15 min präinkubiert. Die Reaktion wurde durch Addition von Substrat (50 µM) gestartet. Das freigesetzte AMC wurde durch Fluoreszenzemission bei 460 nm (Excitation bei 390 nm) unter der Verwendung eines TECAN-Fluorimeters detektiert. Die Aktivität wurde in Fluoreszenz-Einheiten berechnet. Die Inhibierung wird durch IC₅₀-Werte dargestellt.

### Beispiel 4: Zellkultur

HeLa-Zellen wurden in RPMI, supplementiert mit 10% FCS und Penicillin/ Streptomycin, bei 5% CO₂ kultiviert. Inhibitoren wurden von 100x-Vorratslösungen (in DMSO) verabreicht, bei den angegebenen Endkonzentrationen, und mit den Zellen für mindestens 20 Stunden inkubiert.

### Beispiel 5: Intrazelluläre Hemmung von Proteasomen

Alle Peptid-Mimetika **(7-28)** wurden auf ihre Fähigkeit, das 20S Proteasom zu inhibieren, getestet. Dazu wurde zunächst die Inhibierung der löslichen zellulären Proteasen untersucht. 10 µM Lösungen der Verbindungen **7** bis **28** wurden zur zytosolischen Fraktion von HeLa-Zellen addiert und für 30 min auf Eis inkubiert. Nachfolgend wurde der proteolytische Prozess durch Addition des Peptid-Substrats Suc-LLVY-AMC verfolgt. Parallel dazu wurde die cytosolische Fraktion vor der Addition des Substrats mit dem Protease-Inhibitor-Cocktail *Complete* (Roche), der eine breite Spezifität aufweist, behandelt. Dieser Inhibitor-Cocktail beeinflusste die Proteasomenaktivität nicht. 11 der 22 untersuchten Verbindungen verringerten die Proteolyse der zytosolischen Fraktion sowie im *Complete*-vorbehandelten Lysat (Figur 4). Die Inhibierungs-Raten unterschieden sich drastisch. Einige der Verbindungen zeigten keine Inhibierung, wohingegen 5 der analysierten Verbindungen die Hydrolyse von Suc-LLVY-AMC um mehr als 75% verringerten.

Kultivierte Zellen (HeLa) wurden geerntet und mit 0,1% NP40 in TEAD in der Anwesenheit der kommerziell erhältlichen Protease-Inhibitor-Mischung *Complete* (Roche) lysiert. Die proteasomale Aktivität wurde in 10 µl des Lysats unter der Verwendung von Suc-LLVY-AMC als ein Substrat gemessen. Der Proteingehalt wurde mittels Bradford quantifiziert (Protein assay; BioRad).

### Beispiel 6: Spezifität der Verbindungen bei der 20S Proteasom-Inhibierung

Um sicherzustellen, daß der inhibitorische Effekt, der in der zytosolischen Fraktion beobachtet wurde, durch die Inhibierung der Proteasomen verursacht wurde, wurden die Inhibitoren in verschiedenen Konzentrationen zu isolierten 20S Proteasomen addiert. Ihr Effekt wurde mit dem des häufig verwendeten proteasomalen Inhibitors **3** (MG132) verglichen. Die Chymotrypsin-artigen (Suc-LLVY-AMC), die Trypsin-artigen (Bz-VGR-AMC) und die Caspase-artigen (Z-LLE-AMC)-Aktivitäten von 20S Proteasomen wurden nach Inkubation für eine Stunde bei 37°C bestimmt. Die Ergebnisse sind in Tabelle 1 aufgeführt.

Die stärksten inhibitorischen Effekte wurden für die Chymotrypsin-artige Aktivität beobachtet. Sechs der getesteten Inhibitoren (Verbindungen **13, 15, 25, 26, 27, 28**) zeigten IC₅₀-Werte von weniger als 1 µM. Die Inhibierung der Trypsin-artigen Aktivität war niedriger als 1 µM für die Inhibitoren **7, 13** und **15**. Nur Verbindungen **7** und **8** zeigten eine exklusive Inhibierung der Trypsin-artigen Aktivität. Die Inhibierung der Caspase-Aktivität war sogar noch schwächer (siehe Tabelle 1).

Die Proteasomen, die von HeLa-Zellen isoliert werden, beinhalten überwiegend Proteasomen mit konstitutiven Untereinheiten. Daher wiederholten wir die Inhibierungs-Experimente mit Immunproteasomen, die von stabil transfizierten T2.27 Zellen isoliert wurden. Immunproteasomen zeigten eine ähnliche Sensitivität gegenüber unseren Verbindungen (Daten nicht gezeigt).

**Tabelle 1. Berechnete IC₅₀-Werte der Verbindungen 7-28.**

| Verbindung | Nr. der Verbindung | IC₅₀ (µM) | | | |
|---|---|---|---|---|---|
| | | β5 Chymotrypsin-artig (Y) | β5 Chym.-artig (L) | β2 Trypsin-artig (R) | β1 Caspase-artig (E) |
| **7** | BSc2114 | > 10 | - | 0.053 | > 10 |
| **8** | BSc2117 | > 10 | - | 5.481 | > 10 |
| **9** | BSc2207 | > 10 | | - | - |
| **10** | BSc2195 | > 10 | | - | - |
| **11** | BSc2196 | > 10 | | - | - |
| **12** | BSc2194 | > 10 | | - | - |
| **13** | BSc2115 | 0.382 | 0.102 | 0.495 | 0.098 |
| **14** | BSc2128 | >10 | > 10 | >10 | > 10 |
| **15** | BSc2118 | 0.058 | 0.031 | 0.155 | 1.791 |
| **16** | BSc2129 | 7.26 | - | > 10 | > 10 |
| **17** | BSc2208 | - | - | - | - |
| **18** | BSc2197 | 1.731 | | - | 3.122 |
| **19** | BSc2158 | - | - | - | - |
| **20** | BSc2166 | > 10 | | > 10 | > 10 |
| **21** | BSc2167 | 1.303 | > 10 | - | - |
| **22** | BSc2160 | 2.196 | - | - | - |
| **23** | BSc2159 | - | - | - | - |
| **24** | BSc2185 | - | - | - | - |
| **25** | BSc2186 | 0.981 | | - | 4.04 |
| **26** | BSc2187 | 0.441 | | - | 1.72 |
| **27** | BSc2188 | 0.350 | | - | 7.966 |
| **28** | BSc2189 | 0.072 | | - | > 10 |
| **3** | MG132 | 0.0242 | 2.240 | 9.215 | 2.288 |

Die IC₅₀-Werte wurden aus der Inhibierung des Proteasoms bei steigenden Konzentrationen des Inhibitors berechnet. Die Proben wurden für 15 min in Eis prä-inkubiert. Der Assay wurde durch Addition von 50 µM eines der folgenden fluorogenen Peptidsubstrate gestartet:
LLVY-MCA und GLL-MCA für Chymotrypsin-artige Aktivität,
VGR-MCA für Trypsin-artige Aktivität
LLE-MCA für Caspase-artige Aktivität.

Die Freisetzung von MCA wurde bei 460 nm Emission (Excitation 390 nm) bestimmt. Die berechneten IC₅₀-Werte für MG132 dienen als Kontrollen.

### Beispiel 7: Sensitivität von Zellen gegenüber den addierten Verbindungen

Protease-Inhibitoren sind oftmals sehr toxisch für Organismen oder einzelne Zellen (1). Daher wurden ausgewählte Inhibitoren in Zellkulturen getestet.

Die Viabilität von Hela-Zellen in Anwesenheit von verschiedenen Verbindungen wurde in 24 Stunden-Kulturen getestet. Die Viabilität von HeLa-Zellen wurde durch Kristallviolett-Färbung nach der Inkubation mit den Inhibitoren getestet. Die Zellen wurden einmal mit PBS gewaschen, mit 1% Glutardialdehyd für 30 min fixiert und erneut gewaschen. Am Ende wurden die fixierten Zellen mit 0,1% Kristallviolett in PBS für 30 min gefärbt und nachfolgend sorgfältig mit Wasser gewaschen, um ungebundenen Farbstoff zu entfernen. Der verbleibende Farbstoff wurde durch 0,1% Triton X-100 in PBS eluiert und bei 550 nm bestimmt.

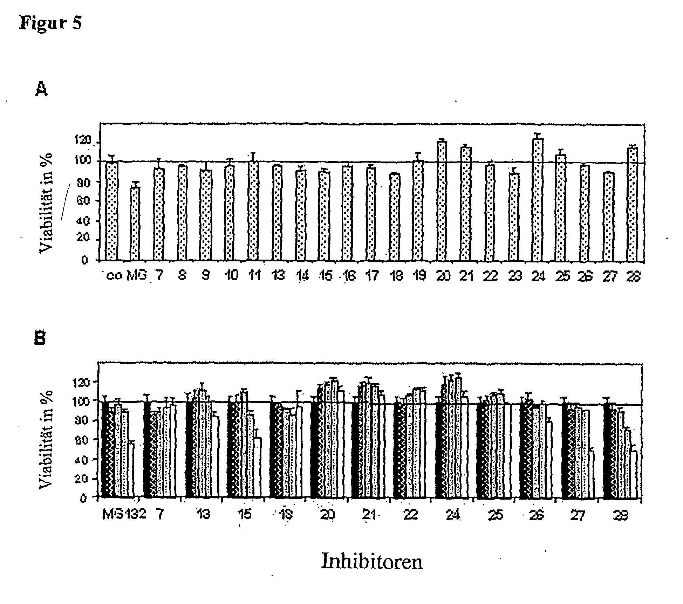

HeLa-Zellen tolerierten 1 µM Konzentrationen von inhibitorischen und inaktiven Substanzen (Figur 5A). Die Überlebensrate war bei 10 µM Lösungen eindeutig verringert, insbesondere für die potentesten Inhibitoren (Verbindungen **15, 28, 27**) (Figur 5B).

Eine Anwendung auf Zellkulturen oder Tiere setzt voraus, daß die Konzentrationen der Inhibitoren so niedrig wie notwendig gewählt werden. Die spezifische proteasomale Aktivität wurde in Zellen, die mit 1 µM der Verbindungen **15, 22, 25, 26**, bzw. **28** behandelt wurden, auf einen Wert unter 50% reduziert (Figur 6A). Eine deutliche Reduktion der spezifischen Aktivität wurde für Verbindungen **7, 13** und **27** beobachtet. Verbindungen **18** und **21** zeigten nur eine sehr schwache Inhibierung der proteasomalen Aktivität (Daten nicht gezeigt). Bemerkenswerterweise wurde eine Reduktion der proteolytischen Aktivität sogar schon bei einer Konzentration von 10 nM der Verbindungen **15, 22** und **28** beobachtet.

### Beispiel 8: Detektion von akkumulierten poly-ubiquitinylierten Proteinen

Eine spezifische Inhibierung der Proteasome führte zur Akkumulation von poly-ubiquitinylierten Proteinen. Tatsächlich stieg die Menge von poly-ubiquitinylierten Proteinen während der Inkubation mit den Inhibitoren an. Erste Effekte wurden nach 2 Stunden beobachtet, und zwar für Verbindung **15** (Figur 6B) sowie für Verbindungen **20, 22, 25** and 28 (Daten nicht gezeigt). Die Ergebnisse für Verbindungen **15, 25, 26, 27** und **28** nach 24 Stunden Inkubation zeigt Figur 6C.

Es wurden 50 µg von Gesamt-Zell-Lysat durch SDS-PAGE separiert und auf PVDF-Membran (Millipore) geblottet. Die Blots wurden mittels 5% einer Milchsuspension geblockt. Die polyubiquitinylierten Proteine wurden durch Anti-Ubiquitin-Antikörper (DAKO) und Anti-Kaninchen, POD-markiert, als sekundärer Antikörper (DIANOVA) detektiert und durch ECL visualisiert.

### Beispiel 9: Apoptose-Assay

Proteasome bestimmen das empfindliche Gleichgewicht zwischen Leben und Tod der Zellen durch Kontrolle der Transkriptionsfaktoren und der Proteine, die in Apoptose involviert sind. Die Reduktion von proteasomaler Aktivität könnte zur Initiierung von Apoptose führen, was für den proteasomalen Inhibitor 3 (MG132) berichtet wurde (29).

Dazu wurden 10,000 HeLa-Zellen pro Well in einer 96-Well-Platte ausgesät und mit 1 µM der Inhibitoren **7, 8, 11, 13-16, 18, 20-23** und **25-28** für 20 Stundenko-kultiviert. Die Induktion der Apoptose wurde durch die Messung der Caspase 3/7-Aktivität (Apo-One^{®}-Assay, Promega) bestimmt.

Die Behandlung von Zellen mit den meisten der Inhibitoren resultierte in einer Reduktion der Zell-Viabilität. Für die Inhibitoren **7, 15, 26** und **28** konnte durch die Aktivierung von Caspase 3 und 7 eine beginnende Apoptose als Ursache für die verminderte Viabilität gezeigt werden (Figur 7).

Ähnliche Ergebnisse wurden durch Fluoreszenzmikroskopie von DAPI-gefärbter Kern-Fragmentation beobachtet (Daten nicht gezeigt).

### Beispiel 10: Tumorzellen zeigen eine höhere Empfindlichkeit gegenüber Inhibitoren

Humane Melanomzellen (MeWo) wurden für 72 h mit verschiedenen Konzentrationen der Inhibitoren **15** und **28** inkubiert und die Viabilität der Zellen mittels Kristallviolett-Färbung bestimmt (Figur 8). Eine Überlebensrate von 50% der Zellen wurde für die Verbindung 15 bei einer Konzentration von 15 nM, im Vergleich dazu **3** (MG132) mit 35 nM, beobachtet. Fibroblasten, die zur Kontrolle mit beiden Verbindungen behandelt wurden, zeigten eine 50%ige Reduktion der Viabilität unter gleichen Bedingungen bei ca. 500- 1000 nM. Ähnliche Resultate wurden für die Verbindung **28** erhalten (Daten nicht gezeigt).

### Beispiel 11: Untersuchungen zum Zellzyklusarrest

Humane Melanomzellen (MeWo) wurden mit den Verbindungen **15** and **3** (MG132) für 24 Stunden ko-kultiviert. Die Zellen wurden mit PBS gewaschen, in 70% Ethanol fixiert und anschließend mit RNAse A behandelt. Die DNA wurde durch Propidiumjodid (5 µg/ml) gefärbt und mittels Flow-Zytometrie analysiert (FACS Calibur flow cytometer; Beckton Dickinson). Die relative Verteilung der Zellen, die sich in verschiedenen Phasen des Zellzyklus befanden, konnte so ermittelt werden. Ein Arrest des Zellzyklus in der G2 Phase konnte bei Anwendung von 50 nM des Inhibitors **15** unter den obigen Bedingungen beobachtet werden. Im Vergleich dazu waren für einen G2-Zyklusarrest in den selben Zellen 100 nM 3 (MG132) erforderlich (Figur 8 B,C). Die statistische Signifikanz wurde durch den Chi-Square- Test ermittelt.

### Beispiel 12: Ko-Kristallisation

Weiterhin wurde die Kristallstruktur des 20S Hefe-Proteasoms in Komplex mit dem Inhibitor **15** bestimmt.

Dazu wurden Kristalle des 20S Proteasoms von *S. cerevisiae* in hängenden Tropfen bei 24°C, wie zuvor beschrieben (6), gezüchtet und für 60 min mit Verbindung **15** inkubiert. Die verwendete Proteinkonzentration für die Kristallisation betrug 40 mg/ml in Tris-HCl (10 mM, pH 7,5) und EDTA (1 mM). Die Tropfen enthielten 3 µl Protein und 2 µl Reservoir-Lösung, enthaltend 30 mM Magnesiumacetat, 100 mM Morpholinethansulfonsäure (pH 7,2) und 10% MPD.

Die Raumgruppe gehört zu P2₁ mit Zelldimensionen von a = 135,8 Å, b = 300,1 Å, c = 144,4 Å und β = 113,1°. Daten für 2,8 Å wurden gesammelt unter der Verwendung von Synchroton-Strahlung mit λ = 1,05 Å an der BW6-Beamline des DESY, Hamburg, Deutschland. Kristalle wurden in einen cryo-schützenden Puffer (30% MPD, 20 mM Magnesiumacetat, 100 mM Morpholinethansulfonsäure pH 6,9) eingeweicht und in einem Strom von flüssigem Stickstoffgas bei 90K gefroren (Oxford Cryo Systems). Röntgenstrahlen-Intensitäten wurden unter Verwendung des MOSFILM Programm-Pakets (Version 6.1) evaluiert und Datenreduktion wurde mit CCP4 (24) durchgeführt. Die Anisotropie der Diffraktion wurde korrigiert durch einen allgemeinen anisotropischen Temperaturfaktor durch Vergleichen der beobachteten und kalkulierten Struktur-Amplituden unter Verwendung des Programms X-PLOR (25). Ein Gesamtzahl von 2383416 Reflexionen, die zu 248616 einzigen (unique) Reflexionen führten (96,9% Vollständigkeit), wurden gesammelt. Das korrespondierende R_{merge} betrug 8,7% bei 2,8 Å Auflösung (41,9% für die letzte Auflösungs-Schale). Elektronendichte wurde verbessert durch Mittelwertbildung und Rücktransformation der Reflexionen 10 mal über der zweifachen, nicht-kristallographischen Symmetrieachse unter Verwendung des Programm-Pakets MAIN (26). Konventionelle kristallographische Festkörper, positionale und Temperaturfaktor-Verfeinerungen (refinements) wurden mit X-PLOR unter Verwendung der Struktur des Hefe-20S-Proteasoms als Anfangsmodell (6) ausgeführt. Für Modellierung wurde das Programm MAIN verwendet. Die Struktur wurde auf einen R-Faktor von 21,7% (freier R-Faktor 24,9%) mit rms-Abweichungen von den Ziel-Werten von 007 Å für Bindungen und 1,30° für Winkel verfeinert (27). Modelling-Experimente wurden unter Verwendung der Koordinaten des Hefe-20S-Proteasoms mit dem Programm MAIN durchgeführt (26).

Die Daten zeigen, daß die Verbindung **15** in einer ähnlichen Orientierung an das Threonin im aktiven Zentrum bindet, wie es für den Calpain-Inhibitor I beobachtet wurde (6). Eine definierte Elektronen-Dichte wurde in allen aktiven Zentren gefunden, was anzeigt, daß die Verbindung **15** keine Spezifität für die Untereinheiten bei hohen Konzentrationen des Inhibitors (10 mM) aufweist. Der funktionale Aldehyd des Inhibitors bildet eine kovalente hemi-acetale Bindung zu dem Thr1O^{γ} aus. Das Peptid-Rückrad von **15** nimmt eine β-Konformation ein, füllt die Lücke zwischen den β-Strängen und generiert eine anti-parallele β-Faltblatt-Struktur (Figur 8). Die Seitenkette des Leucin zeigt in die S1-Tasche, wohingegen die P2-Seitenkette nicht in Kontakt mit dem Protein steht. Die Seitenkette des Leucin in P3 interagiert eng mit den Aminosäuren der benachbarten β-Untereinheit. Im allgemeinen spielen S1 und S3-Spezifitäts-Taschen eine vorherrschende Rolle bei der Inhibitor-Bindung, wie auch in den Kristallstrukturen von 20S Proteasom in Komplex mit Lactacystin (6) und Vinylsulfon (30) beobachtet. Der neutrale Charakter von Met45 in der Untereinheit β5 spielt eine dominante Rolle für die Spezifität dieser Untereinheit. Die kristallgraphischen Daten (Figur 9) zeigen auf, daß die P1-Leu-Seitenkette von Verbindung **15** eine strukturelle Umlagerung von Met45 verursacht. Im Gegensatz zur Kristallstruktur des Proteasoms in Komplex mit Lactacystin, ist Met45 um 3 Å verlagert, was einen Kontakt mit der Leucin-Seitenkette in P1 von Verbindung **15** vermeidet, so daß die S1-Tasche geräumiger wird. Bemerkenswerterweise sind die hydrophobischen Interaktionen zwischen dem Leucin-Rest des Inhibitors und Met45 nur schwach, wodurch die durchschnittlicher Verweilzeit der Verbindung im aktiven Zentrum reduziert wird. Die Spezifität der β1- und β2-Taschen wird durch positive bzw. negative Ladungen definiert, welche Protein-Ligand-Interaktionen destabilisieren. Jedoch verursacht die inhärente Reaktivität des Aldehyds in Verbindung 15 eine Bindung in allen proteolytisch aktiven Zentren. Die funktionale Gruppe dieses Inhibitors übernimmt eine dominante Rolle bei der Bindung.

### Literatur

1. Adams, J.; Proteasome Inhibitors in Cancer Therapy, Humana Press Inc., Totowa, N. J., (2004), p. 77-84.
2. Glickman, M. H., and Ciechanover, A. (2002) Physiological Reviews 82, 373-428
3. Voges, D., Zwickl, P., and Baumeister, W. (1999) Ann. Rev. Biochemistry 68, 1015-1068
4. Peters, J. M., Cejka, Z., Harris, J. R., Kleinschmidt, J. A., and Baumeister, W. (1993) Journal of Molecular Biology 234, 932-937
5. Coux, O., Tanaka, K., and Goldberg, A. L. (1996) Annual Review of Biochemistry 65, 801-847
6. Groll, M., Ditzel, L., Loewe, J., Stock, D., Bochtler, M., Bartunik, H. D., and Huber, R. (1997) Nature 386, 463-471
7. Baumeister, W., Walz, J., Zuhl, F., and Seemuller, E. (1998) Cell 92, 367-380
8. Kloetzel, P.-M., and Ossendorp, F. (2004) Current Opinion in Immunology 16, 76-81
9. Kloetzel, P. M. (2001) Nat. Rev. Mol. Cell Biol. 2, 179-187
10. Serwold T., Gonzalez F., Kim J., Jacob R., Shastri N. (2002) Nature 419, 480-483
11. Seifert, U., Maranon, C., Shmueli, A., Desoutter, J.-F., Wesoloski, L., Janek, K., Henklein, P., Diescher, S., Andrieu, M., de la Salle, H., Weinschenk, T., Schild, H., Laderach, D., Galy, A., Haas, G., Kloetzel, P.-M., Reiss, Y., and Hosmalin, A. (2003) Nature Immunology 4, 375-379
12. Golab, J., Bauer Thomas, M., Daniel, V., and Naujokat, C. (2004) Clinica chimica acta; Int. J. Clin. Chem. 340, 27-40
13. An, B., Goldfarb, R. H., Siman, R., and Dou, Q. P. (1998) Cell Death and Differentiation 5, 1062-1075
14. Orlowski, R. Z., Small, G. W., and Shi, Y. Y. (2002) J. Biol. Chem. 277, 27864-27871
15. Orlowski, R. Z., Eswara, J. R., Lafond-Walker, A., Grever, M. R., Orlowski, M., and Dang, C. V. (1998) Cancer Research 58, 4342-4348
16. Kisselev, A. F., and Goldberg, A. L. (2001) Chemistry & Biology 8, 739-758
17. Groll M., and Huber R. (2004) Biochim. Biophys. Acta 1695, 33-44.
18. Cusack Jr., J. C., Liu, R., Houston, M., Abendroth, K., Elliott, P. J., Adams, J., and Baldwin Jr, A. S. (2001) Cancer Research 61, 3535-3540
19. Orlowski, R. Z., Stinchcombe, T. E., Mitchell, B. S., Shea, T. C., Baldwin, A. S., Stahl, S., Adams, J., Esseltine, D.-L., Elliott, P. J., Pien, C. S., Guerciolini, R., Anderson, J. K., Depcik-Smith, N. D., Bhagat, R., Lehman, M. J., Novick, S. C., O'Connor, O. A., and Soignet, S. L. (2002) J. Clinical Oncology 20, 4420-4427
20. Paramore, A., and Frantz, S. (2003) Nat. Rev. Drug. Discov. 2, 611-612
21. Myung, J., Kim, K. B., and Crews, C. M. (2001) Medicinal Research Reviews 21, 245-273
22. Schmidt, B. (2003) ChemBioChem 4, 366-378
23. John, V., Beck, J. P., Bienkowski, M. J., Sinha, S., and Heinrikson, R. L. (2003) J. Med. Chem. 46, 4625-4630
24. Lesslie, A. G. (1994) MRC Laboratory of Molecular Biology, Cambrige, UK
25. Brunger, A. (1992) *Yale University Press*, New Haven.
26. Turk, D. (1992) Thesis, Technische Universitaet Muenchen
27. Engh, R., and Huber, R. (1991) Acta Cryst. A47, 392-400
28. Schmidt, B., Ehlert, D. K., and Braun, H. A. (2004) Tetrahedron Lett. 45, 1751-1753
29. Guzmen, M. L., Swiderski, C. F., Howard, D. S., Grimes, B. A., Rossi, R. M., Szilvassy, S. J., and Jordan, C. T. (2002) Proc. Natl. Acad. Sci. USA 99, 16220-5
30. Groll, M., Nazif, T., Huber, R., and Bogyo, M. (2002) Chem. Biol. 9, 655-62
31. Ling, Y. H., Liebes, L., Ng, B., Buckley, M., Elliott, P. J., Adams, J., Jiang, J. D., Muggia, F. M., and Perez-Soler, R. (2002) Mol. Cancer Ther. 1, 841-849
32. Meiners, S., Heyken, D., Wellej, A., Ludwig, A., Stangl, K., Kloetzel, P.-M., and Krüger, E. (2003) J. Biol. Chem. 278, 21517-25
33. Adams, J. (2004) Nat. Rev. Cancer 4, 349-360
34. Kisselev, A. F., and Goldberg, A. L. (2001) Chem. Biol. 8,739-58
35. Traenker, E. B., Wilk, S., and Baeuerle, P. A. (1994) EMBO J. 13, 5433-41
36. Stoklosa, T., Golab, J., Wojcik, C., Wlodarski, P., Jalili, A., Januszko, P., Giermasz, A., Wilczynski, G. M., Pleban, E., Marczak, M., Wilk, S., and Jakobisiak, M. (2004) Apoptosis 9, 193-204
37. Hideshima, T., Richardson, P., Chauhan, D., Palombella, V. J., Elliott, P. J., Adams, J., and Anderson, K. C. (2001) Cancer Res. 61, 3071-3076
38. Garcia-Echeverria, C., Imbach, P., France, D., Fürst, P., Lang, M., Noorani, A. M., Scholz, D., Zimmermann, J., and Furet, P. (2001) Bioorg Med Chem Lett. 11, 1317-1319.
39. Prösch, S. et al. (2003) Antiviral Therapy 8:555-67.
40. Lou, H. et al. (2003) Am J Pathol. 163:381-85).
41. Golab, J. et al. (2004) Clin.Chim.Acta 340:27-40.

## Patentansprüche

1. Verbindung mit der Formel 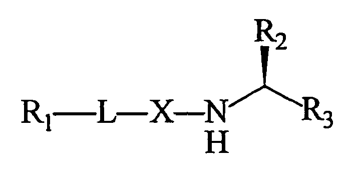 worin
R₁ Boc, Z, Ac oder H ist,
L Leu ist,
X Asp(OR₄) ist,
R₂ CH₂-CH(CH₃)₂ ist,
R₃ CH₂-OH, CH=O, CH(OH)-C≡C-Phenyl, CH(OH)-C(O)-NH-R₅ oder C(O)-(O)-NH-R₅ ist,
R₄ *t*-Butyl, Benzyl oder H ist,
R₅ Benzyl, 3-Picolyl oder Phenyl ist,
und pharmazeutisch akzeptable Salze davon.

2. Verbindung nach Anspruch 1, worin
R₁ Boc oder Z,
L Leu,
X Asp(OR₄),
R₂ CH₂-CH(CH₃)₂,
R₃ CH₂-OH,
R₄ *t*-Butyl,
ist.

3. Verbindung nach Anspruch 1, worin
R₁ Boc, Z oder Ac,
L Leu,
X Asp(OR₄),
R₂ CH₂-CH(CH₃)₂,
R₃ CH=O,
R₄ *t*-Butyl oder Benzyl,
ist.

4. Verfahren zur Herstellung einer Verbindung nach Anspruch 1, umfassend die Kondensation von herkömmlich geschützten Dipeptiden und Aminosäuren mit Aminoalkoholen und anschließender Oxidation zu Peptidaldehyden.

5. Verfahren nach Anspruch 4, **dadurch gekennzeichnet, dass** die Oxidation unter der Verwendung von hypervalenten Iodreagentien und insbesondere IBX in DMSO erfolgt.

6. Verbindung nach einem der Ansprüche 1 bis 3 zur Behandlung von Erkrankungen.

7. Pharmazeutische Zusammensetzung, umfassend eine Verbindung nach einem der Ansprüche 1 bis 3 oder ein pharmazeutisch akzeptables Salz davon sowie pharmazeutisch akzeptable Träger und/oder Hilfsstoffe.

8. Pharmazeutische Zusammensetzung nach Anspruch 7, **dadurch gekennzeichnet, daß** die Verbindung in einer Menge vorliegt, so daß ein Konzentrationsbereich von 0,001 bis 100 µM bei der Behandlung *in vivo* vorliegt.

9. Pharmazeutische Zusammensetzung nach Anspruch 7 oder 8, **dadurch gekennzeichnet, daß** die Verbindung in einer Menge vorliegt, die effektiv die Proteasom-Funktion in einer Zelle oder einem Säugetier hemmt.

10. Verwendung einer Verbindung nach einem der Ansprüche 1 bis 3 zur Herstellung eines Medikaments zur Induktion von Apoptose in Zellen.

11. Verwendung einer Verbindung nach einem der Ansprüche 1 bis 3 zur Herstellung eines Medikaments zur Inhibierung der proteolytischen Aktivität von 20S Proteasom, 26S Proteasom und Immunoproteasom.

12. Verwendung nach Anspruch 11, wobei spezifisch die Trypsin-artige Aktivität von 20S Proteasom und 26S Proteasom inhibiert wird.

13. Verwendung nach Anspruch 11, wobei spezifisch die Chymotrypsin-artige Aktivität von 20S Proteasom und 26S Proteasom inhibiert wird.

14. Verwendung nach Anspruch 11, wobei gleichzeitig die Chymotrypsin-artigen, Trypsin-artigen und Caspase-artigen Aktivitäten von 20S Proteasom und 26S Proteasom inhibiert werden.

15. Ex vivo-Verfahren zur Inhibierung des Wachstums einer Krebszelle, umfassend in Kontakt bringen einer Zelle mit einer Verbindung nach einem der Ansprüche 1 bis 3 oder mit einer pharmazeutischen Zusammensetzung nach einem der Ansprüche 7 bis 9.

## Claims

1. A compound of formula 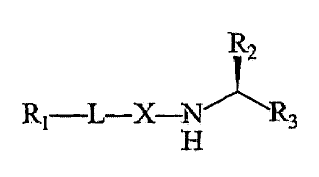 wherein
R₁ is Boc, Z, Ac or H,
L is Leu,
X is Asp(OR₄),
R₂ is CH₂-CH(CH₃)₂,
R₃ is CH₂-OH, CH=O, CH(OH)-C≡C phenyl, CH(OH)-C(O)-NH-R₅ or C(O)- C(O)-NH-R₅,
R₄ is *t*-butyl, benzyl or H,
R₅ is benzyl, 3-picolyl or phenyl,
and pharmaceutically acceptable salts thereof.

2. The compound according to claim 1, wherein
R₁ is Boc or Z,
L is Leu,
X is Asp(OR₄),
R₂ is CH₂-CH(CH₃)₂,
R₃ is CH₂-OH,
R₄ is *t*-butyl.

3. The compound according to claim 1, wherein
R₁ is Boc, Z or Ac,
L is Leu,
X is Asp(OR₄),
R₂ is CH₂-CH(CH₃)₂,
R₃ is CH=O,
R₄ is *t*-butyl or benzyl.

4. A method for preparing a compound according to claim 1, comprising condensation of conventionally protected dipeptides and amino acids with amino alcohols and subsequent oxidation to give peptide aldehydes.

5. The method according to claim 4, **characterized in that** the oxidation is carried out using hypervalent iodine reagents, and in particular IBX in DMSO.

6. A compound according to one of claims 1 to 3 for the treatment of diseases.

7. A pharmaceutical composition, comprising a compound according to one of claims 1 to 3 or a pharmaceutically acceptable salt thereof and pharmaceutically acceptable carriers and/or excipients.

8. The pharmaceutical composition according to claim 7, **characterized in that** the compound is present at an amount such that the concentration ranges from 0.001 to 100 µM during *in vivo* treatment.

9. The pharmaceutical composition according to claim 7 or 8, **characterized in that** the compound is present at an amount which effectively inhibits the proteasome function in a cell or a mammalian.

10. Use of a compound according to one of claims 1 to 3 for preparing a medicament for inducing apoptosis in cells.

11. The use of a compound according to one of claims 1 to 3 for preparing a medicament for inhibiting the proteolytic activity of 20S proteasome, 26S proteasome and immunoproteasome.

12. The use according to claim 11, wherein the trypsin-like activity of 20S proteasome and 26S proteasom is specifically inhibited.

13. The use according to claim 11, wherein the chymotrypsin-like activity of 20S proteasom and 26S proteasome is specifically inhibited.

14. The use according to claim 11, wherein the chymotrypsin-like, trypsin-like, and caspase-like activities of 20S proteasome and 26S proteasome are simultaneously inhibited.

15. An *ex vivo* method for inhibiting the growth of a cancer cell, comprising contacting a cell and a compound according to one of claims 1 to 3, or a cell and a pharmaceutical composition according to one of claims 7 to 9.

## Revendications

1. Composé de formule 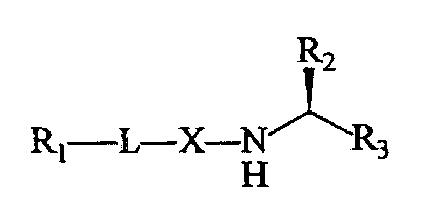 dans laquelle
R₁ est Boc, Z, Ac ou H,
L est Leu,
X est Asp(OR₄),
R₂ est CH₂-CH(CH₃)₂,
R₃ est CH₂-OH, CH=O, CH(OH)-C≡C-phényle,
CH(OH)-C(O)-NH-R₅ ou C(O)-C(O)-NH-R₅,
R₄ est un groupe t-butyle, benzyle ou H,
R₅ est un groupe benzyle, 3-picolyle ou phényle, et leurs sels pharmaceutiquement acceptables.

2. Composé selon la revendication 1, dans lequel
R₁ est Boc ou Z,
L est Leu,
X est Asp(OR₄),
R₂ est CH₂-CH(CH₃)₂,
R₃ est CH₂-OH,
R₄ est un groupe t-butyle.

3. Composé selon la revendication 1, dans lequel
R₁ est Boc, Z ou Ac,
L est Leu,
X est Asp(OR₄),
R₂ est CH₂-CH(CH₃)₂,
R₃ est CH=O,
R₄ est un groupe t-butyle ou benzyle.

4. Procédé de production d'un composé selon la revendication 1, comprenant la condensation de dipeptides protégés de façon habituelle et d'acides aminés avec des amino-alcools et ensuite, l'oxydation en peptidaldéhydes.

5. Procédé selon la revendication 4, **caractérisé en ce que** l'oxydation s'effectue en utilisant des réactifs iodés hypervalents et en particulier, IBX dans du DMSO.

6. Composé selon l'une des revendications 1 à 3, pour le traitement de maladies.

7. Composition pharmaceutique comprenant un composé selon l'une des revendications 1 à 3 ou un sel pharmaceutiquement acceptable de celui-ci et des véhicules ou adjuvants pharmaceutiquement acceptables.

8. Composition pharmaceutique selon la revendication 7, **caractérisée en ce que** le composé est présent en une quantité telle que l'on ait une plage de concentrations de 0,001 à 100 µM lors du traitement *in vivo*.

9. Composition pharmaceutique selon la revendication 7 ou 8, **caractérisée en ce que** le composé se présente en une quantité qui inhibe efficacement la fonction du protéasome dans une cellule ou chez un mammifère.

10. Utilisation d'un composé selon l'une des revendications 1 à 3, pour la production d'un médicament pour l'induction de l'apoptose dans des cellules.

11. Utilisation d'un composé selon l'une des revendications 1 à 3, pour la production d'un médicament destiné à inhiber l'activité protéolytique du 20S protéasome, du 26S protéasome et de l'immunoprotéasome.

12. Utilisation selon la revendication 11, dans laquelle l'activité de type trypsine du 20S protéasome et du 26S protéasome est inhibée spécifiquement.

13. Utilisation selon la revendication 11, dans laquelle l'activité de type chymotrypsine du 20S protéasome et du 26S protéasome est inhibée spécifiquement.

14. Utilisation selon la revendication 11, dans laquelle les activités de type chymotrypsine, de type trypsine et de type caspase du 20S protéasome et du 26S protéasome sont inhibées en même temps.

15. Procédé *ex vivo* d'inhibition de la croissance d'une cellule cancéreuse, comprenant la mise en contact d'une cellule avec un composé selon l'une des revendications 1 à 3, ou avec une composition pharmaceutique selon l'une des revendications 7 à 9.
